# EUROPEAN PATENT APPLICATION

(11) **EP 3 502 102 A1**
(43) Date of publication of application: **26.06.2019**
(21) Application number: 17209072.2
(22) Date of filing: 20.12.2017
(51) Int. Cl.: C07D 307/71, A61P 25/00, A61P 9/00, A61P 29/00, A61P 35/00, A61P 37/00, A61P 31/12

(54) **STING INHIBITORS**

(71) Applicant: ECOLE POLYTECHNIQUE FEDERALE DE LAUSANNE (EPFL), 1015 Lausanne (CH)
(72) Inventor: ABLASSER, Andrea, 1091 Aran (CH); REYMOND, Luc, 1806 ST-LEGIER (CH); HAAG, Simone, 1026 ECHANDENS-DENGES (CH)
(74) Representative: KATZAROV S.A.

(57) **Abstract**

The present invention relates to compounds, compositions for use and methods for the treatment of a STING associated disease, condition, or disorder.

## Description

### FIELD OF THE INVENTION

The present invention relates to compounds, compositions for use and methods for the treatment of a STING associated disease, condition, or disorder.

### BACKGROUND OF THE INVENTION

STING (Stimulator of Interferon Genes) is an innate signalling molecule that is pivotal in mediating the recognition of cytosolic DNA ^{1,2}. In this context, STING, a transmembrane protein localized to the endoplasmic reticulum (ER), acts as a second messenger receptor for 2', 3' cyclic GMP-AMP (hereafter cGAMP), which is produced by cGAS after dsDNA binding³⁻⁷. In addition, STING can also function as a primary pattern recognition receptor for bacterial cyclic dinucleotides (CDNs) and small molecule agonists ^{8,9,10}. The recognition of endogenous or prokaryotic CDNs proceeds through the carboxy-terminal domain of STING, which faces into the cytosol and creates a V-shaped binding pocket formed by a STING homodimer ¹¹⁻¹⁴. Ligand-induced activation of STING triggers its re-localization to the Golgi, a process essential to promote the interaction of STING with TBK1¹⁵. This protein complex, in turn, signals through the transcription factors IRF-3 to induce type I interferons (IFNs) and other co-regulated antiviral factors¹⁶. In addition, STING was shown to trigger NF-• B and MAP kinase activation ^{17,18}. Following the initiation of signal transduction, STING is rapidly degraded, a step considered important in terminating the inflammatory response¹⁹.

Excessive activation of STING is associated with a subset of monogenic autoinflammatory conditions, the so-called type I interferonopathies²⁰. Examples of these diseases include a clinical syndrome referred to as STING-associated vasculopathy with onset in infancy (SAVI), which is caused by gain-of-function mutations in TMEM173 (the gene name of STING) ^{21,22}. Moreover, STING is implicated in the pathogenesis of Aicardi-Goutieres Syndrome (AGS) and genetic forms of lupus ²³⁻²⁵. As opposed to SAVI, it is the dysregulation of nucleic acid metabolism that underlies continuous innate immune activation in AGS. Apart from these genetic disorders, emerging evidence points to a more general pathogenic role for STING in a range of inflammation-associated disorders such as systemic lupus erythematosus, rheumatoid arthritis and cancer ^{26,27}. Thus, small molecule-based pharmacological interventions into the STING signalling pathway hold significant potential for the treatment of a wide spectrum of diseases. However, direct STING antagonists have to our best knowledge not previously been described. Here we identify and characterise two covalent small molecule inhibitors of STING, C-178 and C-176, that are highly selective and bear potent activity both *in vitro* and *in vivo.*

### BRIEF DESCRIPTION OF THE FIGURES

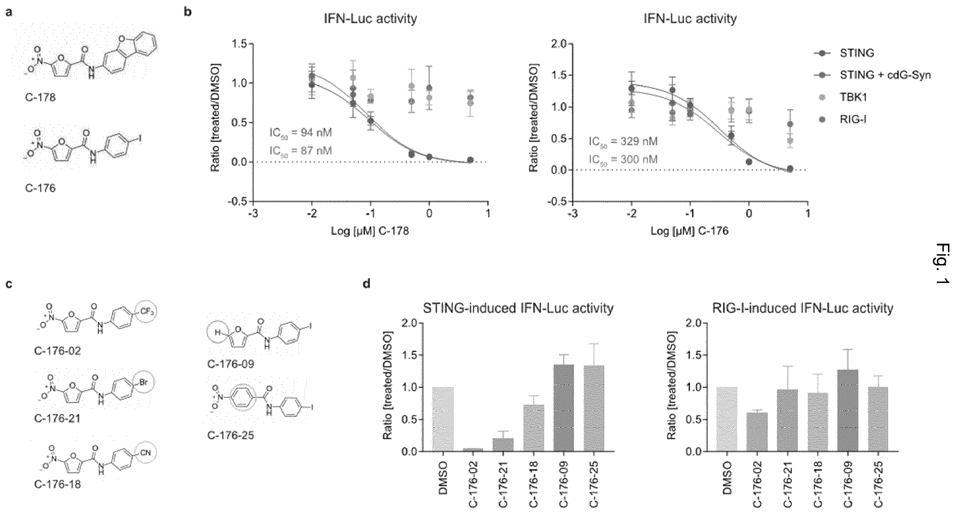

**Figure 1** **| A high-throughput chemical screen identifies two small molecule inhibitors of the STING signalling pathway.**
   **a,** Chemical structure of C-178 and C-176. b, HEK293T cells were transfected with vectors encoding for STING, STING in combination with cyclic di-GMP synthase (cdG-Syn), TBK1 or RIG-I and a IFN-• luciferase reporter, respectively, and treated with C-178 (left panel) or C-176 (right panel) (concentration of the compounds: 0.01-5µM). Reporter activity was measured 16 h after compound treatment. A nonlinear regression analysis was performed. Concentrations of C-178 and C-168 are shown in Log values. **c,** Chemical structures of synthesized derivatives of C-168 are shown. Circles indicate substitutes compared to C-176. **d, e,** Chemical compounds from (c) were tested at 0.5 µM for inhibitory activity in HEK293T cells transfected with STING (**e**) or RIG-I (**f**) in conjunction with IFN-• luciferase reporter, respectively. **b, e, f,** Values of inhibitor-treated cells were normalized to DMSO-treated cells. Data are shown as mean ± s.e.m of three independent experiments carried out in duplicates.

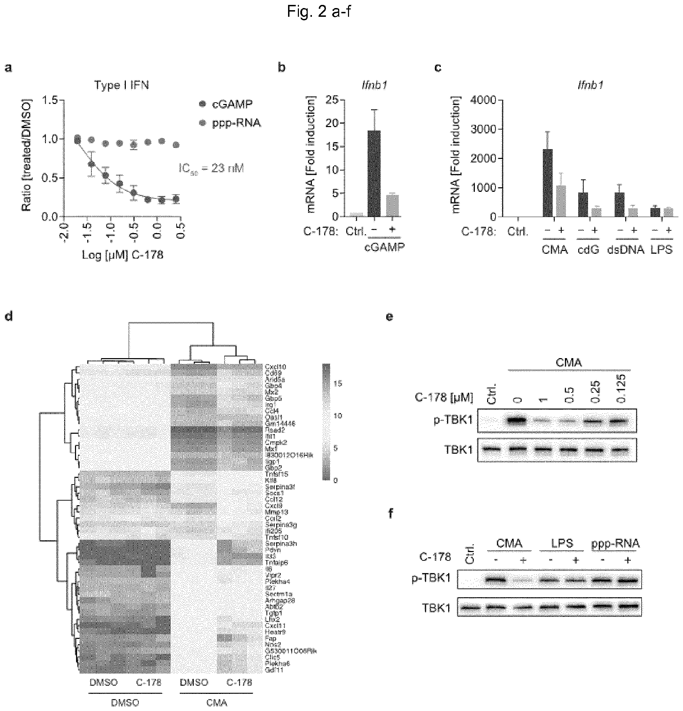

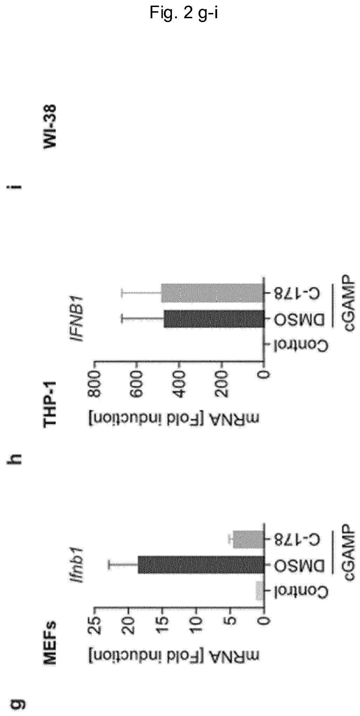

**Figure 2** **| Inhibitory activity of C-178 in primary cells.**
   **a,** Production of type I IFNs of bone marrow-derived macrophages (BMDMs) stimulated with cGAMP or triphosphate RNA after pre-treatment with C-178 (0.02 µM- 2.5 µM) or DMSO as measured by a type I IFN bioassay. Cytokine levels were normalized to DMSO-treated control cells. A nonlinear regression analysis was performed for cGAMP stimulation. **b, c,** BMDMs were pre-treated with C-178 (0.25 µM) and stimulated with cGAMP, cyclic di-GMP, CMA, 90mer dsDNA or LPS for 3 h. The induction of *Ifnb1* mRNA levels was assessed by RT-qPCR. **d,** Heatmaps of RNA sequencing analysis of BMDMs treated with DMSO or CMA and with or without addition of C-178. Top 50 up-regulated genes in DMSO-treated versus CMA-treated cells without C-178 are shown. (**e**) Immunoblotting of phospho-TBK1 (p-TBK1) and TBK1 of BMDMs after pre-treatment with C-178 for 1 h and stimulation with CMA as indicated. **f,** Immunoblots of p-TBK1 and TBK1 obtained from BMDMs exposed to C-178 and stimulated with CMA, LPS or triphosphate RNA or left untreated are shown. **g, h, i,** Mouse embryonic fibroblasts (MEFs) (**g**), THP-1 cells (**h**) and WI-38 cells (**i**) were treated with C-178 (0.25 µM) for 1 h and stimulated with cGAMP. The induction of mRNA levels of *Ifnb1*/*IFNB1* were measured by qRT-PCR. **a, b, c, g, h, i,** Mean ± s.e.m of three independent experiments carried out in duplicates is shown. **e, f,** One representative experiment of three independent experiments is shown.

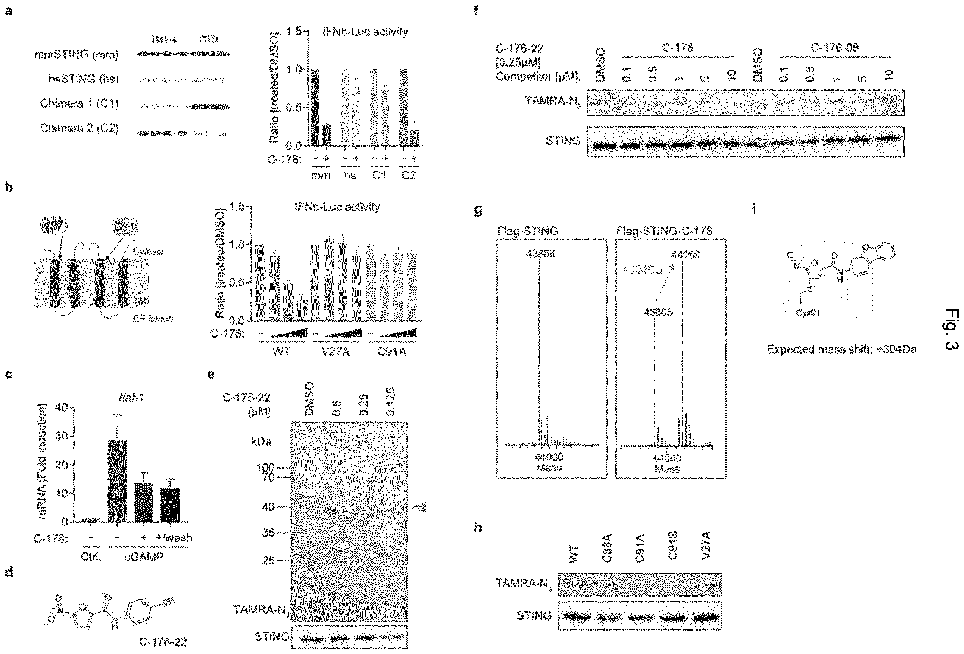

**Figure 3** **| Irreversible inhibition of STING by covalent binding of C-178 to cysteine 91 (C91).**
   **a, b,** HEK293T cells were transfected with the indicated constructs (chimeric construct 1 (C1): hsSTING(AA1-138)-mmSTING(AA138-378); chimeric construct 2 (C2): mmSTING(AA1-137)-hsSTING(AA139-379) in conjunction with cyclic di-GMP synthase, IFN-• luciferase reporter and treated with C-178 (0.5 µm) (**a**) or C-178 (0.31-0.07 µM) (**b**). Reporter activity was measured 16 h after transfection and values were normalized to DMSO-treated control cells. **c,** BMDMs were treated with C-178 (0.25 µM) for 1 h, washed or left untreated and after an additional hour stimulated with cGAMP. The induction of *Ifnb1* mRNA levels was assessed by RT-qPCR after 3h. **d,** Chemical structure of C-176-22. **e,** Labelling of recombinant STING in HEK293T cells with C-176-22 in indicated concentrations. **f,** Concentration-dependent competitor blockage of C-176-22 against the active compound C-178 and the inactive compound C-176-09 performed in HEK293T cells expressing mmSTING. **g,** Deconvoluted ESI mass spectrum showing intact mass of Flag-STING (left) and the Flag-STING-C-176 complex (right). **h,** Labelling of indicated STING constructs in HEK293T cells with C-176-22 (0.25 µM). **i,** Proposed modification of C-178 on STING C91. **a, b, d,** Mean ± s.e.m of three independent experiments carried out in duplicates is shown. **e, f, g, h,** One representative experiment of three independent experiments is shown.

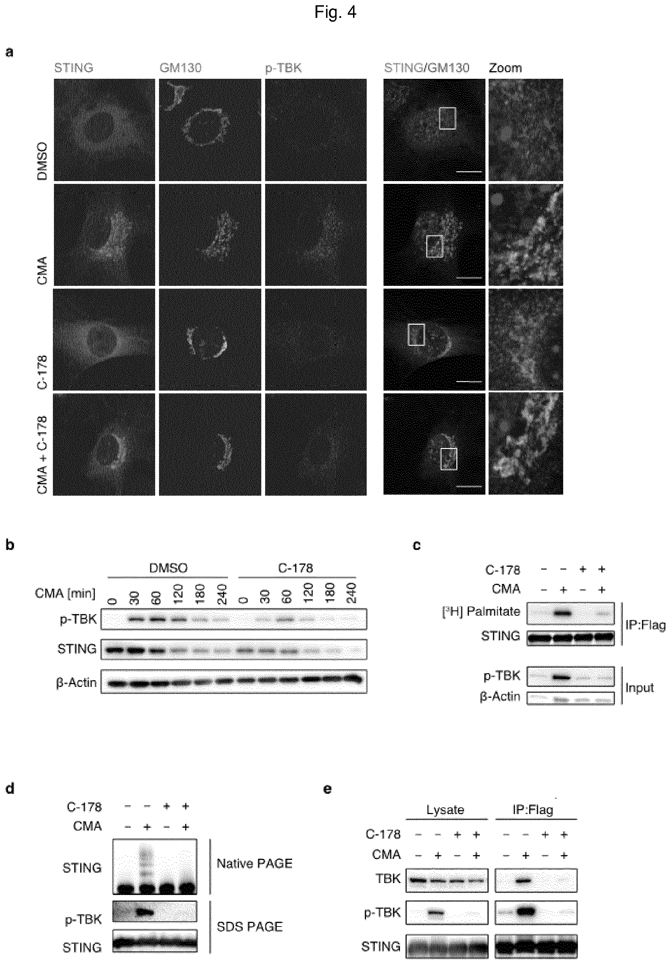

**Figure 4** **| C-178 blocks activation-induced palmitoylation of STING and prevents recruitment of TBK1.**
   **a,** MEFs were transduced with Flag-STING, treated as indicated (C-178 (1.5 µM)), stimulated with CMA, fixed, permeabilised and stained for Flag, GM130, and p-TBK1. Nuclei were stained with DAPI. Scale bar 20 µm. **b,** Immunoblots of p-TBK1, STING or•-Actin from cell lysates of MEFs treated with C-178 (1 µM) or DMSO and stimulated with CMA for indicated periods. **c,** HEK293T cells expressing Flag-STING were starved for 1h, treated with C-178 (1 µM) or DMSO and incubated with [³H] palmitate, followed by a stimulation with CMA for 2 h. Flag-STING was immunoprecipitated from whole cell lysate and analysed by autoradiography. In parallel, cell lysates were analysed by immunoblotting for p-TBK1 and STING. **d,** HEK293T cells expressing STING were treated as indicated (C-178 (1 µM)) and stimulated with CMA for 1.5 h. Whole cell lysates were analysed by blue native PAGE (upper panel) and SDS-PAGE (lower panels) followed by immunoblotting of STING and p-TBK1. **e,** HEK293T cells expressing Flag-STING were pre-treated as indicated (C-178 (1 µM)) and stimulated with CMA for 2 h. Flag-immunoprecipitation (IP) was carried out in whole cell lysate and co-precipitated proteins detected by immunoblotting. **a-e,** One representative experiment out of three independent experiments is shown.
**Figure 5 | C-176 is active *in vivo* and attenuates disease features of Trex1 KO mice.**
   **a,** Serum levels of type I IFNs (left panel) and IL-6 (right panel) from C57BL/6 mice pre-treated with C-168 or vehicle control as measured by bioassay (type I IFN) and ELISA (IL-6) 4 h after CMA treatment. Data shown are mean ± s.e.m of *n* = 3 mice per condition; ** *P* < 0.01 by One-way ANOVA test. **b,** MEFs obtained from WT mice or Trex1 KO mice were treated with DMSO or C-178 (2 µM) overnight. Depicted genes were measured by RT-qPCR. Data are shown as mean ± s.e.m of *n* = 2 distinct MEF cultures (WT MEFs) or *n* = 7 distinct MEF cultures (Trex1 KO MEFs); *** *P* < 0.001 by One-way ANOVA test. **c, d, e, Trex1** heterozygous mice treated with vehicle (n = 6) or **Trex1** KO mice were treated with C-168 (*n* = 5) or vehicle (*n* = 5) for 11 days. Histological analysis of the heart and measurement of serum levels of type I IFNs are shown. * *P* < 0.05, ** *P* < 0.01, *** *P* < 0.001, **** *P* < 0.0001 as measured by One-way ANOVA test.

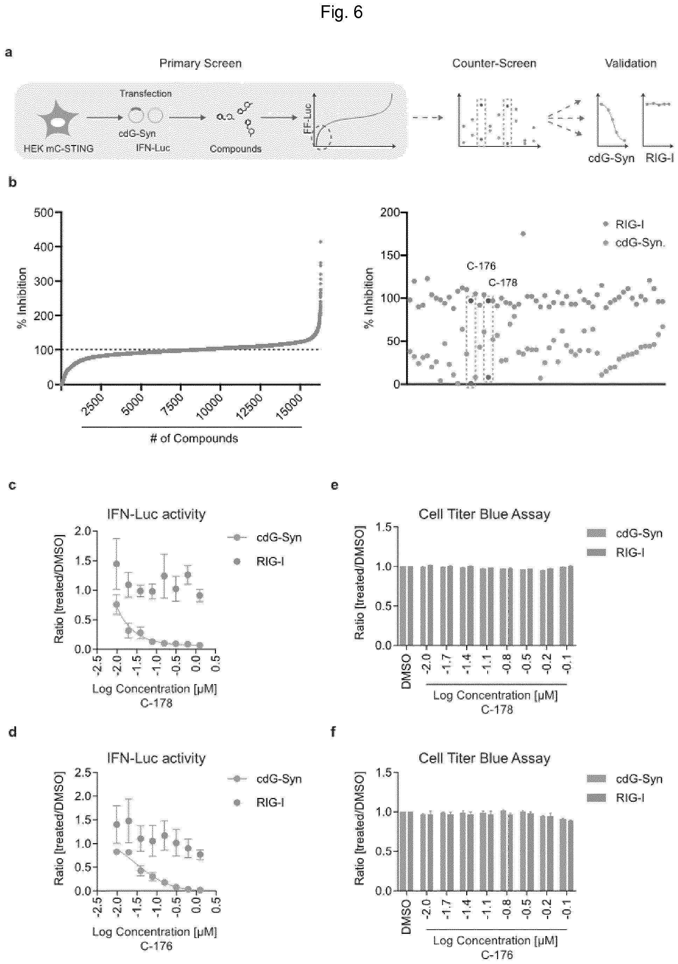

**Figure 6** **| A high-throughput chemical screen identifies two small molecule inhibitors of the STING signalling pathway.**
   **a,** Screening workflow for the identification of small molecules that target the STING signalling pathway (Hek mC-STING = HEK293T cells expressing mCherry-STING) **b,** Left panel: summary of the primary screen. Data points represent the mean of normalised values for IFN-• luciferase activity of compound-treated cells versus DMSO-treated control cells. Right panel: Selected hit candidates from the primary screen were validated in a secondary screen. Normalised values for cdG-Syn/STING-induced IFN-• luciferase activity (light blue) are shown in comparison to those obtained for RIG-I-triggered activity (green). Compounds C-178 and C-176 are highlighted in black. Mean of duplicate measurements is shown. **c-f,** HEK293T cells stably expressing mCherry-STING were transfected with plasmids encoding for cdG-Syn or RIG-I in conjunction with an IFN-• luciferase reporter. After transfection, cells were treated with C-178 (**c, e**) or C-176 (**d, f**) in concentrations ranging from 1.25 µM - 0.009 µM and 16 h later luciferase activity (**c, d**) was determined or cell viability was measure by CellTiter-Blue assay (**e, f**). Mean + s.e.m of three independent experiments carried out in duplicates is shown.
**Figure 7** **| C176L is inhibitory active.**
   HEK293T cells were transfected with mmSTING in conjunction with an IFN-• luciferase reporter and treated with C-176-22 in indicated concentration. 16 h later Luciferase activity was measured. Mean + s.e.m of two independent experiments measured in duplicates is shown. DMSO-treated control cells.
**Figure 8** **| C176L highly specifically labels mmSTING.**
   **a,** HEK293T cells and HEK293T cells expressing STING were labelled with C-176-22 (0.25µM), clicked to a TAMRA azide and the whole cell lysate was loaded on an SDS-PAGE for in-gel fluorescence scanning and immunoblot analysis. **b,** Labelling of mmSTING and hsSTING by C-176-22 expressed in HEK293T cells. One representative experiments of two independent experiments is shown.

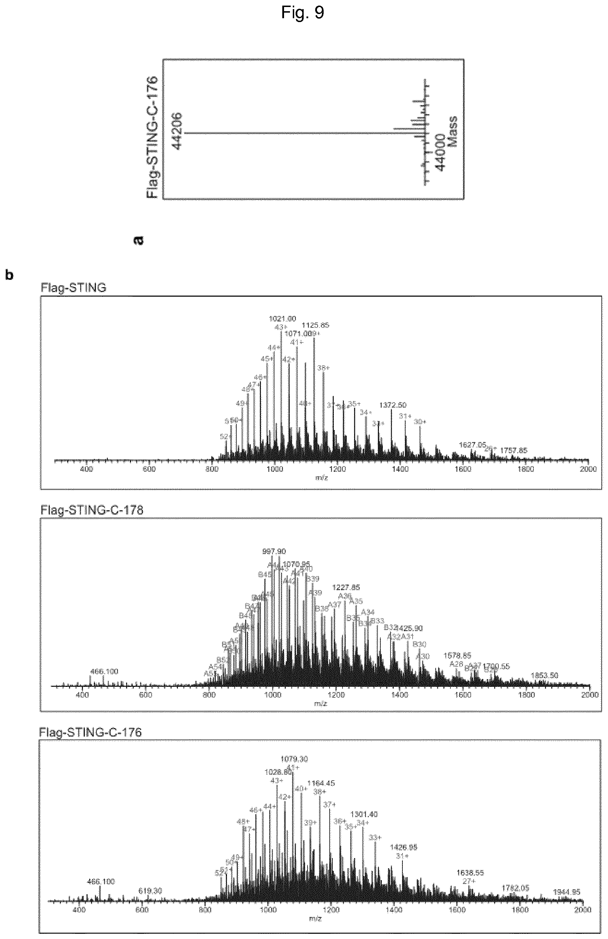

**Figure 9** **| ESI and deconvoluted ESI spectra of STING, STING-C-176 and STING-C-178.**
   **a, b,** HEK293T cells expressing Flag-STING were treated with C-178 or C-176 (1µM), lysed and STING was immunoprecipitated, eluted and protein mass spectrometry was performed. Deconvoluted ESI mass spectrum shows intact mass of the Flag-STING-C-176 (**a**), complex and ESI spectra of Flag-STING (**b,** upper panel), Flag-STING-C178 (**b,** middle panel) and Flag-STING-C-176 (**b,** lower panel). One representative experiments of two independent experiments is shown.

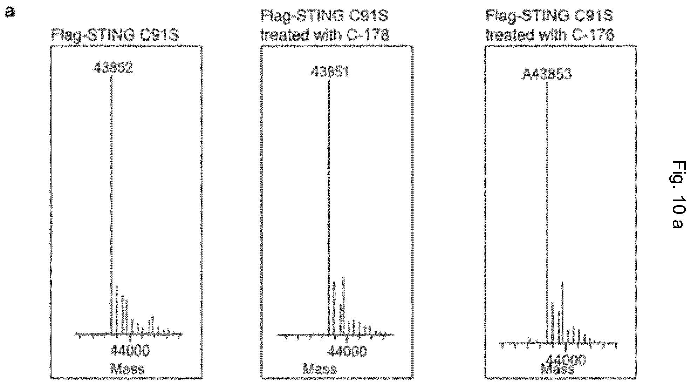

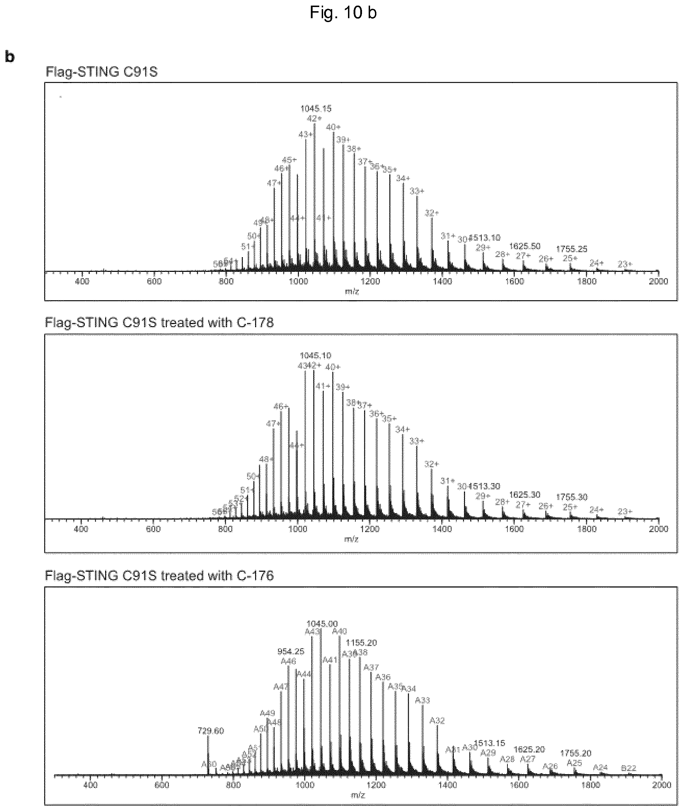

**Figure 10** **| ESI and deconvoluted spectra of STING C91S.**
   **a,** HEK293T cells expressing Flag-STING C91S were treated with C-178 or C-176 (1µM), lysed and STING was immunoprecipitated, eluted and protein mass spectrometry was performed. Deconvoluted ESI mass spectrum showing intact mass of the Flag-STING C91S (**a,** left panel), Flag-STING C91S treated with C-178 (**a,** middle panel) and Flag-STING C91S treated with C-168 (a, right panel). **b,** ESI mass spectrum of Flag-STING C91S (**b,** upper panel), Flag-STING C91S treated with C-178 (**b,** middle panel) and Flag-STING C91S treated with C-176 (**b,** lower panel). One representative experiments of two independent experiments is shown.

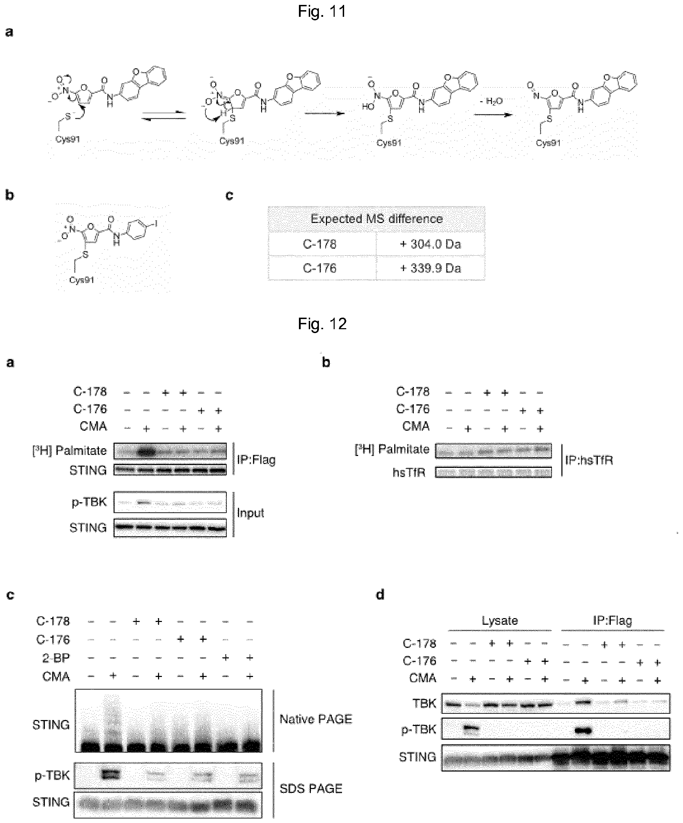

**Figure 11** **| Mechanism of covalent binding.**
   **a,** Proposed "nitrobenzyl rearrangement-like" mechanism for the covalent modification of cysteine 91 by C-178. **b,** Product of the covalent binding of C-176 to cysteine 91. **c,** calculated mass shift of C-178 and C-176 after covalent binding to STING.
**Figure 12** **| C-178 and C-176 block activation-induced palmitoylation of STING and recruitment of TBK1.**
   **a,** HEK293T cells expressing Flag-STING were treated as indicated C-178 and C-176 (1 µM), starved for 1 h, followed by incubation with [³H] palmitate together with CMA for 2 h. Flag-STING was immunoprecipitated and analysed by autoradiography. Cell lysate was analysed by immunoblotting. **b,** Palmitoylation of the endogenous human transferrin receptor was analysed from cell lysates described in (**a**). **c,** HEK293T cells expressing STING were treated as indicated with C-178, C-176 (1 µM), 2-BP (50 µM) and stimulated with CMA for 1.5 h. Whole cells lysates were loaded on a blue native PAGE or SDS-PAGE and indicated proteins were analysed by western blotting, **d,** HEK293T cells expressing Flag-STING were treated as indicated with C-178 and C-176 (1 µM) and stimulated with CMA for 2 h. Flag-STING was immunoprecipitated from whole cell extracts and coprecipitated proteins were visualized by immunoblotting. One representative experiments of three independent experiments is shown.

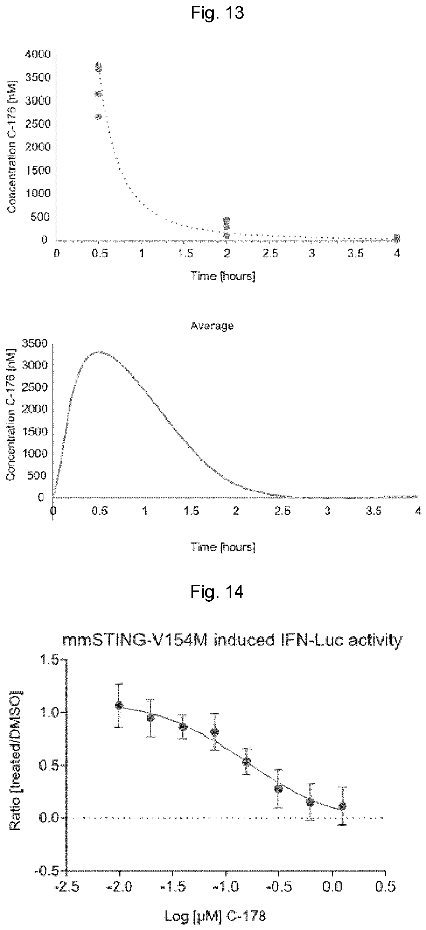

**Figure 13** **| *In vivo* pharmacokinetics of C-176.**
   Mean plasma concentration profiles of C-176 following a single dose intraperitoneal injection into C57BL/6 mice (*n* = 3 mice per time point).
**Figure 14** **| C-178 blocks type I IFN responses triggered by gain-of-function STING mutants.**
   HEK293T cells were transfected with plasmids encoding for mmSTING-V154M together with an IFN-• luciferase reporter. After transfection, cells were treated with C-178 in concentrations ranging from 1.25 µM - 0.009 µM and 16 h later luciferase activity was determined. Mean ± s.e.m of three independent experiments carried out in duplicates is shown.

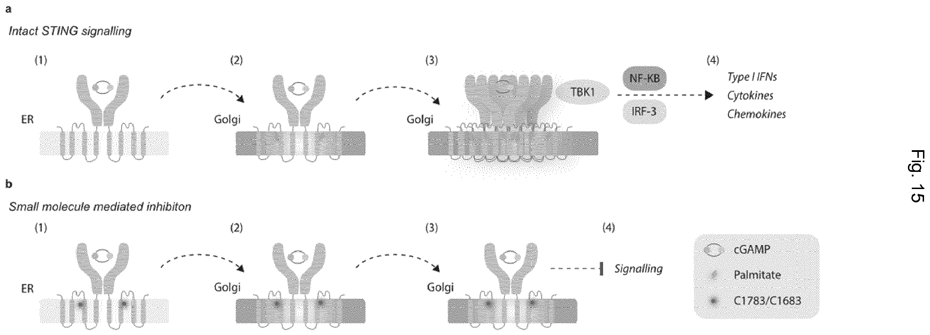

**Figure 15** **| Model of mechanism of action of C-178 and C-176.**
   **a,** Under normal conditions, activated STING translocates to the Golgi, where it is palmitoylated on membrane proximal cysteine residues, including cysteine 91. Palmitoylation facilitates the assembly of STING into high molecular weight complexes providing a platform for the recruitment of and interaction with TBK1 and, possibly, other adaptor molecules. This, in turn, result in the activation of transcription factors, including NF-• B and IRF-3, to induce the expression of cytokines, chemokines and type I interferons. **b,** Following treatment with C-178 or C-176, STING is irreversibly modified at cysteine 91. Ligand-induced activation of STING under these inhibitory conditions triggers its translocation to the Golgi, however, STING cannot undergo palmitoylation and, consequently, does not form clusters. As a result, STING is kept in an "inactive" state, unable to recruit TBK1 and, thus, to induce downstream signalling.

### SUMMARY OF THE INVENTION

The present invention provides compounds presenting formula (I): 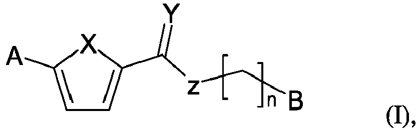 racemates, enantiomers, diastereomers and pharmaceutically acceptable salts thereof, wherein
A is selected from the group consisting of -NO₂, -C(O)OH, -C(O)O-, -C(O)OR¹,-C(O)R¹, -C(O)NH₂, -C(O)NHR¹, -C(O)NR²R³, -S(O)R¹, -SO₂R¹, -S(O)₂NH₂, -S(O)₂NHR¹,-S(O)₂NR²R^{3,} -S(O)(NR¹)R², -P(O)(OH)₂, -P(O)OHR¹, and -P(O)OR¹R²;
R¹, R² and R³ are each independently selected from H, halogen, C₁-C₆ alkyl, C₂-C₆ alkenyl, C₁-C₆ haloalkyl, C₃-C₇ cycloalkyl, -OH, -OR₄, -SH, -SR⁴, -NH₂, -NHR⁴ and -N(R⁴)₂, wherein R⁴ is C₁-C₆ alkyl optionally substituted with halogen, -OH, -CN, -NH₂ or =O;
or R² and R³ together with the nitrogen atom to which they are attached form a substituted or unsubstituted 4-7 membered heterocycle;
X and Z are each independently selected from O, S, or N;
Y is O or S;
B is selected from the group consisting of 6-31 membered aryl; 6-31 membered heteroaryl, 5-31 membered aryloxy, 5-31 membered aralkyl, optionally substituted with 1, 2, 3 substituents independently selected from -O-CF₃, -CF₃, -O-CHF₂, halogen, -OH, -NH₂,-CN, -OCH₃, C₁-C₆ alkyl, allyl, -C(O)OR¹, -C(O)NH₂, propargyl, -NO₂, -S(O)₂NH₂, and-S(O)CH₃; and
n is an integer between 0-2.

Preferably, the compounds of formula I of the invention will have the following substituents,
i) A is -NO₂; or
ii) X is O; Y is O; or
iii) A is -NO₂, X is O; Y is O; or
iv) A is -NO₂, X is O; Y is O; and Z is N, or
v) B is -CF₃, halogen, -CN, or propargyl,
or any combinations between i), ii), iii), iv) and v).

A further object of the present invention is to provide compound having formula I, or racemate, enantiomer, diastereomer and pharmaceutically acceptable salt thereof, for use in the treatment of a STING associated disease.

A further object of the present invention concerns a pharmaceutical composition comprising a compound of the invention having formula I, or a pharmaceutically acceptable salt thereof, and a pharmaceutically acceptable excipient.

The present invention also concerns the use of a compound of the invention having formula I, or pharmaceutical composition of claim, in the manufacture of a medicament. Preferably, the medicament is for the treatment of a STING associated disease.

The present invention further concerns a compound that inhibits the signaling molecule STING by binding at least one amino acid of a region including the amino acid residues 85 to 95. Preferably, the binding is a covalent binding.

A kit comprising (1) a composition comprising a pharmaceutically effective amount of a of the invention having formula I or racemates, enantiomers, diastereomers and pharmaceutically acceptable salts thereof; and (2) instructions for use is also envisioned.

### DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The present application provides compounds that inhibit the signaling molecule STING by binding at least one amino acid of a region including the amino acid residues 85 to 95. The binding can be covalent or non-covalent. Preferably, the binding is a covalent binding. Most preferably, the compound covalently binds to the transmembrane-located cysteine, C91.
By acting through, preferably, covalent binding to a transmembrane-located cysteine, C91, the compounds of the invention exploit an unanticipated mechanism in order to attain potency and selectivity against STING. In the absence of inhibition, ligand binding triggers the translocation of STING to the Golgi, where palmitoylation occurs at luminal proximal cysteine residues (C91 and C88)^{15 31}. This post-translational modification, in turn, facilitates the multimerisation of STING creating a platform for the recruitment of TBK1 - and possibly other adaptor molecules - and thereby enabling the initiation of further downstream signalling ^{29 16} . Through covalent interaction with C91, the compounds of the invention and in particular C-178 and C-176 block palmitoylation of STING and retain the protein in a signalling incompetent state. Of major significance, the palmitate-modified cysteines are highly conserved residues. This provides confidence that the mechanistic insight of small molecule-mediated antagonism of STING delineated here will be useful for the development of next-generation inhibitors of STING that are active in humans. For now, C-178 and C-176 provide a powerful new tool to acutely perturb STING *in vitro* and offer the unique possibility to pharmacologically intervene into the function of STING *in vivo.* Along these considerations, previous studies using genetic approaches have uncovered the detrimental role of STING in the pathogenesis of certain monogenic autoinflammatory disorders ^{20 36}. This has led to the widespread idea that inhibiting STING may be a *key route* in combating these diseases. However, the lack of molecules targeting STING has so far precluded testing this hypothesis experimentally. The work presented here provides compelling evidence that indeed drug-mediated suppression of STING is effective in the setting of inflammatory disease. The present study proves for the first time the feasibility to exploit anti-STING drugs for the treatment of AGS and SAVI.

The compounds of the invention are preferably compounds of formula (I): 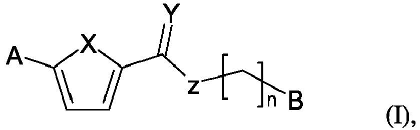 racemates, enantiomers, diastereomers and pharmaceutically acceptable salts thereof, wherein
A is selected from the group consisting of -NO₂, -C(O)OH, -C(O)O-, -C(O)OR¹,-C(O)R¹, -C(O)NH₂, -C(O)NHR¹, -C(O)NR²R³, -S(O)R¹, -SO₂R¹, -S(O)₂NH₂, -S(O)₂NHR¹,-S(O)₂NR²R^{3,} -S(O)(NR¹)R², -P(O)(OH)₂, -P(O)OHR¹, and -P(O)OR¹R²;
R¹, R² and R³ are each independently selected from H, halogen, C₁-C₆ alkyl, C₂-C₆ alkenyl, C₁-C₆ haloalkyl, C₃-C₇ cycloalkyl, -OH, -OR₄, -SH, -SR⁴, -NH₂, -NHR⁴ and -N(R⁴)₂, wherein R⁴ is C₁-C₆ alkyl optionally substituted with halogen, -OH, -CN, -NH₂ or =O;
or R²and R³ together with the nitrogen atom to which they are attached form a substituted or unsubstituted 4-7 membered heterocycle;
X and Z are each independently selected from O, S, or N;
Y is O or S;
B is selected from the group consisting of 6-31 membered aryl; 6-31 membered heteroaryl, 5-31 membered aryloxy, 5-31 membered aralkyl, optionally substituted with 1, 2, 3 substituents independently selected from -O-CF₃, -CF₃, -O-CHF₂, halogen, -OH, -NH₂,-CN, -OCH₃, C₁-C₆ alkyl, allyl, -C(O)OR¹, -C(O)NH₂, propargyl, -NO₂, -S(O)₂NH₂, and-S(O)CH₃; and
n is an integer between 0-2.

Preferably, the compounds of formula I of the invention will have the following substituents,
i) A is -NO₂; or
ii) X is O; Y is O; or
iii) A is -NO₂, X is O; Y is O; or
iv) A is -NO₂, X is O; Y is O; and Z is N, or
v) B is -CF₃, halogen, -CN, or propargyl,
or any combinations between i), ii), iii), iv) and v).

More preferably, the compound of formula I of the invention is selected from the group consisting in 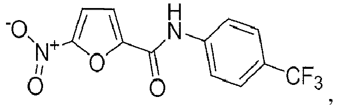 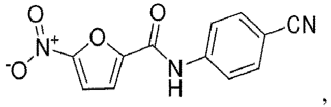 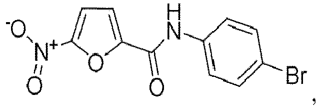 and

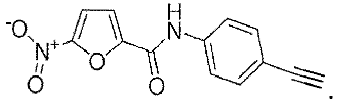

The present invention also concerns a compound of the invention having formula I, or a racemate, enantiomer, diastereomer and pharmaceutically acceptable salt thereof, for use in the treatment of a STING associated disease.

Also envisioned is a pharmaceutical composition comprising a compound of the invention having formula I, or a pharmaceutically acceptable salt thereof, and a pharmaceutically acceptable excipient.

The present invention also considers the use of a compound of the invention having formula I, or pharmaceutical composition of claim, in the manufacture of a medicament. Preferably, the medicament is for the treatment of a STING associated disease.

The present invention also considers a compound that inhibits the signaling molecule STING by binding at least one amino acid of a region including the amino acid residues 85 to 95. Preferably, the binding is a covalent binding. Most preferably, the compound of the invention inhibits the signaling molecule STING by covalently binding amino acid residue 91 (CYS91).

A kit comprising (1) a composition comprising a pharmaceutically effective amount of a of the invention having formula I or racemates, enantiomers, diastereomers and pharmaceutically acceptable salts thereof; and (2) instructions for use is also envisioned.

The term "comprise/comprising" is generally used in the sense of include/including, that is to say permitting the presence of one or more features or components. The terms "comprise" and "comprising" also encompass the more restricted ones "consist" and "consisting", respectively.

As used in the specification and claims, the singular form "a", "an" and "the" include plural references unless the context clearly dictates otherwise.

As used herein the terms "subject", or " patient" are well-recognized in the art, and, are used interchangeably herein to refer to a mammal, including dog, cat, rat, mouse, monkey, cow, horse, goat, sheep, pig, camel, and, most preferably, a human. In some embodiments, the subject is a subject in need of treatment or a subject suffering from a STING associated disease. However, in other aspects, the subject can be a normal subject. The term does not denote a particular age or sex. Thus, adult and newborn subjects, whether male or female, are intended to be covered.

Reference throughout this specification to "one aspect" "an aspect" "another aspect," "a particular aspect," combinations thereof means that a particular feature, structure or characteristic described in connection with the invention aspect is included in at least one aspect of the present invention. Furthermore, the particular features, structures, or characteristics may be combined in any suitable manner in one or more aspects.

The term "treatment" or "treating" means any administration of a composition, pharmaceutical composition, compound, etc... of the disclosure to a subject for the purpose of:
(i) preventing the disease, condition, or disorder, that is, causing the clinical symptoms of the disease not to develop;
(ii) inhibiting the disease, condition, or disorder, that is, arresting the development of clinical symptoms; and/or
(iii) relieving the disease, condition, or disorder, that is, causing the regression of clinical symptoms.

In the context of the present invention, the disease, condition, or disorder is a STING associated disease, condition, or disorder that is alleviated by treatment with a compound, composition, or pharmaceutical composition, of the disclosure that inhibits the signaling molecule STING.
In some aspects, the condition, disease or disorder is cancer. Non-limiting examples of cancer include melanoma, carcinoma, lymphoma, blastoma, sarcoma, and leukemia or lymphoid malignancies. More particular examples of such cancers include breast cancer, colon cancer, rectal cancer, colorectal cancer, kidney or renal cancer, clear cell cancer lung cancer including small-cell lung cancer, non- small cell lung cancer, adenocarcinoma of the lung and squamous carcinoma of the lung, squamous cell cancer (e.g. epithelial squamous cell cancer), cervical cancer, ovarian cancer, prostate cancer, prostatic neoplasms, liver cancer, bladder cancer, cancer of the peritoneum, hepatocellular cancer, gastric or stomach cancer including gastrointestinal cancer, gastrointestinal stromal tumor, pancreatic cancer, head and neck cancer, glioblastoma, retinoblastoma, astrocytoma, thecomas, arrhenoblastomas, hepatoma, hematologic malignancies including non-Hodgkins lymphoma (NHL), multiple myeloma, myelodysplasia disorders, myeloproliferative disorders, chronic myelogenous leukemia, and acute hematologic malignancies, endometrial or uterine carcinoma, endometriosis, endometrial stromal sarcoma, fibrosarcomas, choriocarcinoma, salivary gland carcinoma, vulval cancer, thyroid cancer, esophageal carcinomas, hepatic carcinoma, anal carcinoma, penile carcinoma, nasopharyngeal carcinoma, laryngeal carcinomas, Kaposi's sarcoma, mast cell sarcoma, ovarian sarcoma, uterine sarcoma, melanoma, malignant mesothelioma, skin carcinomas, Schwannoma, oligodendroglioma, neuroblastomas, neuroectodermal tumor, rhabdomyosarcoma, osteogenic sarcoma, leiomyosarcomas, Ewing Sarcoma, peripheral primitive neuroectodermal tumor, urinary tract carcinomas, thyroid carcinomas, Wilm's tumor, as well as abnormal vascular proliferation associated with phakomatoses, edema (such as that associated with brain tumors), and Meigs' syndrome. In some cases, the cancer is melanoma.
In some aspects, the condition, disease or disorder is a neurological disorder, which includes disorders that involve the central nervous system (brain, brainstem and cerebellum), the peripheral nervous system (including cranial nerves), and the autonomic nervous system (parts of which are located in both central and peripheral nervous system).
Non-limiting examples of neurological disorder include acquired epileptiform aphasia; acute disseminated encephalomyelitis; adrenoleukodystrophy; age-related macular degeneration; agenesis of the corpus callosum; agnosia; Aicardi syndrome; Alexander disease; Alpers' disease; alternating hemiplegia; Alzheimer's disease; Vascular dementia; amyotrophic lateral sclerosis; anencephaly; Angelman syndrome; angiomatosis; anoxia; aphasia; apraxia; arachnoid cysts; arachnoiditis; Anronl-Chiari malformation; arteriovenous malformation; Asperger syndrome; ataxia telegiectasia; attention deficit hyperactivity disorder; autism; autonomic dysfunction; back pain; Batten disease; Behcet's disease; Bell's palsy; benign essential blepharospasm; benign focal; amyotrophy; benign intracranial hypertension; Binswanger's disease; blepharospasm; Bloch Sulzberger syndrome; brachial plexus injury; brain abscess; brain injury; brain tumors (including glioblastoma multiforme); spinal tumor; Brown-Sequard syndrome; Canavan disease; carpal tunnel syndrome; causalgia; central pain syndrome; central pontine myelinolysis; cephalic disorder; cerebral aneurysm; cerebral arteriosclerosis; cerebral atrophy; cerebral gigantism; cerebral palsy; Charcot-Marie-Tooth disease; chemotherapy-induced neuropathy and neuropathic pain; Chiari malformation; chorea; chronic inflammatory demyelinating polyneuropathy; chronic pain; chronic regional pain syndrome; Coffin Lowry syndrome; coma, including persistent vegetative state; congenital facial diplegia; corticobasal degeneration; cranial arteritis; craniosynostosis; Creutzfeldt-Jakob disease; cumulative trauma disorders; Cushing's syndrome; cytomegalic inclusion body disease; cytomegalovirus infection; dancing eyes-dancing feet syndrome; Dandy-Walker syndrome; Dawson disease; De Morsier's syndrome; Dejerine-Klumke palsy; dementia; dermatomyositis; diabetic neuropathy; diffuse sclerosis; dysautonomia; dysgraphia; dyslexia; dystonias; early infantile epileptic encephalopathy; empty sella syndrome; encephalitis; encephaloceles; encephalotrigeminal angiomatosis; epilepsy; Erb's palsy; essential tremor; Fabry's disease; Fahr's syndrome; fainting; familial spastic paralysis; febrile seizures; Fisher syndrome; Friedreich's ataxia; fronto-temporal dementia and other "tauopathies"; Gaucher's disease; Gerstmann's syndrome; giant cell arteritis; giant cell inclusion disease; globoid cell leukodystrophy; Guillain-Barre syndrome; HTLV-1-associated myelopathy; Hallervorden-Spatz disease; head injury; headache; hemifacial spasm; hereditary spastic paraplegia; heredopathia atactica polyneuritiformis; herpes zoster oticus; herpes zoster; Hirayama syndrome; HIV-associated dementia and neuropathy (also neurological manifestations of AIDS); holoprosencephaly; Huntington's disease and other polyglutamine repeat diseases; hydranencephaly; hydrocephalus; hypercortisolism; hypoxia; immune-mediated encephalomyelitis; inclusion body myositis; incontinentia pigmenti; infantile phytanic acid storage disease; infantile refsum disease; infantile spasms; inflammatory myopathy; intracranial cyst; intracranial hypertension; Joubert syndrome; Kearns-Sayre syndrome; Kennedy disease Kinsbourne syndrome; Klippel Feil syndrome; Krabbe disease; Kugelberg-Welander disease; kuru; Lafora disease; Lambert-Eaton myasthenic syndrome; Landau-Kleffner syndrome; lateral medullary (Wallenberg) syndrome; learning disabilities; Leigh's disease; Lennox-Gustaut syndrome; Lesch-Nyhan syndrome; leukodystrophy; Lewy body dementia; Lissencephaly; locked-in syndrome; Lou Gehrig's disease (i.e., motor neuron disease or amyotrophic lateral sclerosis); lumbar disc disease; Lyme disease-neurological sequelae; Machado-Joseph disease; macrencephaly; megalencephaly; Melkersson-Rosenthal syndrome; Menieres disease; meningitis; Menkes disease; metachromatic leukodystrophy; microcephaly; migraine; Miller Fisher syndrome; mini-strokes; mitochondrial myopathies; Mobius syndrome; monomelic amyotrophy; motor neuron disease; Moyamoya disease; mucopolysaccharidoses; milti-infarct dementia; multifocal motor neuropathy; multiple sclerosis and other demyelinating disorders; multiple system atrophy with postural hypotension; p muscular dystrophy; myasthenia gravis; myelinoclastic diffuse sclerosis; myoclonic encephalopathy of infants; myoclonus; myopathy; myotonia congenital; narcolepsy; neurofibromatosis; neuroleptic malignant syndrome; neurological manifestations of AIDS; neurological sequelae of lupus; neuromyotonia; neuronal ceroid lipofuscinosis; neuronal migration disorders; Niemann-Pick disease; O'Sullivan-McLeod syndrome; occipital neuralgia; occult spinal dysraphism sequence; Ohtahara syndrome; olivopontocerebellar atrophy; opsoclonus myoclonus; optic neuritis; orthostatic hypotension; overuse syndrome; paresthesia; Parkinson's disease; paramyotonia congenital; paraneoplastic diseases; paroxysmal attacks; Parry Romberg syndrome; Pelizaeus-Merzbacher disease; periodic paralyses; peripheral neuropathy; painful neuropathy and neuropathic pain; persistent vegetative state; pervasive developmental disorders; photic sneeze reflex; phytanic acid storage disease; Pick's disease; pinched nerve; pituitary tumors; polymyositis; porencephaly; post-polio syndrome; postherpetic neuralgia; postinfectious encephalomyelitis; postural hypotension; Prader-Willi syndrome; primary lateral sclerosis; prion diseases; progressive hemifacial atrophy; progressive multifocal leukoencephalopathy; progressive sclerosing poliodystrophy; progressive supranuclear palsy; pseudotumor cerebri; Ramsay-Hunt syndrome (types I and II); Rasmussen's encephalitis; reflex sympathetic dystrophy syndrome; Refsum disease; repetitive motion disorders; repetitive stress injuries; restless legs syndrome; retrovirus-associated myelopathy; Rett syndrome; Reye's syndrome; Saint Vitus dance; Sandhoff disease; Schilder's disease; schizencephaly; septo-optic dysplasia; shaken baby syndrome; shingles; Shy-Drager syndrome; Sjögren's syndrome; sleep apnea; Soto's syndrome; spasticity; spina bifida; spinal cord injury; spinal cord tumors; spinal muscular atrophy; Stiff-Person syndrome; stroke; Sturge-Weber syndrome; subacute sclerosing panencephalitis; subcortical arteriosclerotic encephalopathy; Sydenham chorea; syncope; syringomyelia; tardive dyskinesia; Tay-Sachs disease; temporal arteritis; tethered spinal cord syndrome; Thomsen disease; thoracic outlet syndrome; Tic Douloureux; Todd's paralysis; Tourette syndrome; transient ischemic attack; transmissible spongiform encephalopathies; transverse myelitis; traumatic brain injury; tremor; trigeminal neuralgia; tropical spastic paraparesis; tuberous sclerosis; vascular dementia (multi-infarct dementia); vasculitis including temporal arteritis; Von Hippel-Lindau disease; Wallenberg's syndrome; Werdnig-Hoffman disease; West syndrome; whiplash; Williams syndrome; Wildon's disease; and Zellweger syndrome.
In some aspects, the condition, disease or disorder is an autoimmune diseases, e.g. a cytosolic DNA-triggered autoinflammatory disease. Non-limiting examples include rheumatoid arthritis, systemic lupus erythematosus, multiple sclerosis, inflammatory bowel diseases (IBDs) comprising Crohn disease (CD) and ulcerative colitis (UC), which are chronic inflammatory conditions with polygenic susceptibility. In certain aspects, the condition is an inflammatory bowel disease. In certain aspects, the condition is Crohn's disease, autoimmune colitis, iatrogenic autoimmune colitis, ulcerative colitis, colitis induced by one or more chemotherapeutic agents, colitis induced by treatment with adoptive cell therapy, colitis associated by one or more alloimmune diseases (such as graft-vs-host disease, e.g., acute graft vs. host disease and chronic graft vs. host disease), radiation enteritis, collagenous colitis, lymphocytic colitis, microscopic colitis, and radiation enteritis. In certain of these aspects, the condition is alloimmune disease (such as graft-vs-host disease, e.g., acute graft vs. host disease and chronic graft vs. host disease), celiac disease, irritable bowel syndrome, rheumatoid arthritis, lupus, scleroderma, psoriasis, cutaneous T-cell lymphoma, uveitis, and mucositis (e.g., oral mucositis, esophageal mucositis or intestinal mucositis).
In some aspects, modulation of the immune system by STING provides for the treatment of diseases, including diseases caused by foreign agents. Exemplary infections by foreign agents which may be treated and/or prevented by the method of the present invention include an infection by a bacterium (e.g., a Gram-positive or Gram-negative bacterium), an infection by a fungus, an infection by a parasite, and an infection by a virus. In one aspect of the present invention, the infection is a bacterial infection (e.g., infection by E. coli, Klebsiella pneumoniae, Pseudomonas aeruginosa, Salmonella spp., Staphylococcus aureus, Streptococcus spp., or vancomycin-resistant enterococcus). In another aspect, the infection is a fungal infection (e.g. infection by a mould, a yeast, or a higher fungus). In still another embodiment, the infection is a parasitic infection (e.g., infection by a single-celled or multicellular parasite, including Giardia duodenalis, Cryptosporidium parvum, Cyclospora cayetanensis, and Toxoplasma gondiz). In yet another aspect, the infection is a viral infection (e.g., infection by a virus associated with AIDS, avian flu, chickenpox, cold sores, common cold, gastroenteritis, glandular fever, influenza, measles, mumps, pharyngitis, pneumonia, rubella, SARS, and lower or upper respiratory tract infection (e.g., respiratory syncytial virus)).
In some aspects, the condition, disease or disorder is selected from the non-limiting group comprising hepatitis B, cardiovascular diseases (including e.g. myocardial infarction) and age-related macular degeneration.
Preferably, the condition, disease or disorder is selected from the group comprising a cancer, a neurological disorder, an autoimmune disease, hepatitis B, uveitis, cardiovascular disease, age-related macular degeneration and mucositis.

The term "pharmaceutical" as used herein refers to a chemical substance intended for use in the cure, treatment, or prevention of disease. Details on techniques for formulation and administration of such compositions may be found in Remington, The Science and Practice of Pharmacy 21st Edition (Mack Publishing Co., Easton, Pa.) and Nielloud and Marti-Mestres, Pharmaceutical Emulsions and Suspensions: 2nd Edition (Marcel Dekker, Inc, New York).
For the purposes of this disclosure, the pharmaceutical compositions may be administered by a variety of means including orally, parenterally, by inhalation spray, topically, or rectally in formulations containing pharmaceutically acceptable carriers, adjuvants and vehicles. The term parenteral as used here includes but is not limited to subcutaneous, intravenous, intramuscular, intraarterial, intradermal, intrathecal and epidural injections with a variety of infusion techniques. Intraarterial and intravenous injection as used herein includes administration through catheters. Administration via intracoronary stents and intracoronary reservoirs is also contemplated. The term oral as used herein includes, but is not limited to oral ingestion, or delivery by a sublingual or buccal route. Oral administration includes fluid drinks, energy bars, as well as pill formulations
Pharmaceutical compositions may be in any form suitable for the intended method of administration. When used for oral use for example, tablets, troches, lozenges, aqueous or oil suspensions, dispersible powders or granules, emulsions, hard or soft capsules, syrups or elixirs may be prepared. Compositions intended for oral use may be prepared according to any method known to the art for the manufacture of pharmaceutical compositions and such compositions may contain one or more agents including sweetening agents, flavoring agents, coloring agents and preserving agents, in order to provide a palatable preparation. Tablets containing a drug compound in admixture with non-toxic pharmaceutically acceptable excipient which are suitable for manufacture of tablets are acceptable. These excipients may be, for example, inert diluents, such as calcium or sodium carbonate, lactose, calcium or sodium phosphate; granulating and disintegrating agents, such as maize starch, or alginic acid; binding agents, such as starch, gelatin or acacia; and lubricating agents; such as magnesium stearate, stearic acid or talc. Tablets may be uncoated, or may be coated by known techniques including enteric coating, colonic coating, or microencapsulation to delay disintegration and adsorption in the gastrointestinal tract and/or provide a sustained action over a longer period. For example, a time delay material such as glyceryl monostearate or glyceryl distearate alone or with a wax may be employed.
Formulations for oral use may be also presented as hard gelatin capsules where the drug compound is mixed with an inert solid diluent, for example calcium phosphate or kaolin, or as soft gelatin capsules wherein the active ingredient is mixed with water or an oil medium, such as peanut oil, liquid paraffin or olive oil.
Such excipients include a suspending agent, such as sodium carboxymethylcellulose, methylcellulose, hydroxypropyl methylcellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycetanol), a condensation product of ethylene oxide with a partial ester derived from a fatty acid and a hexitol anhydride (e.g., polyoxyethylene sorbitan monooleate). The aqueous suspension may also contain one or more preservatives such as ethyl or n-propyl p-hydroxy-benzoate, one or more coloring agents, one or more flavoring agents and one or more sweetening agents, such as sucrose or saccharin.
Oil suspensions may be formulated by suspending the active ingredient in a vegetable oil, such as arachis oil, olive oil, sesame oil or coconut oil, or a mineral oil such as liquid paraffin. The oral suspensions may contain a thickening agent, such as beeswax, hard paraffin or cetyl alcohol. Sweetening agents, such as those set forth above, and flavoring agents may be added to provide a palatable oral preparation. These compositions may be preserved by the addition of an antioxidant such as ascorbic acid.
Dispersible powders and granules of the disclosure suitable for preparation of an aqueous suspension by the addition of water provide the active ingredient in admixture with a dispersing or wetting agent, a suspending agent, and one or more preservatives. Suitable dispersing or wetting agents and suspending agents are exemplified by those disclosed above. Additional excipients, for example sweetening, flavoring and coloring agents, may also be present.
The pharmaceutical compositions of the disclosure may also be in the form of oil-in-water emulsions. The oily phase may be a vegetable oil, such as olive oil or arachis oil, a mineral oil, such as liquid paraffin, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan monooleate, and condensation products of these partial esters with ethylene oxide, such as polyoxyethylene sorbitan monooleate. The emulsion may also contain sweetening and flavoring agents.
Syrups and elixirs may be formulated with sweetening agents, such as glycerol, sorbitol or sucrose. Such formulations may also contain a demulcent, a preservative, a flavoring or a coloring agent.
The pharmaceutical compositions of the disclosure may be in the form of a sterile injectable preparation, such as a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent such as a solution in 1,3-butane-diol or prepared as a lyophilized powder. Among the acceptable vehicles and solvents that may be employed are water, Ringer's solution and isotonic sodium chloride solution. In addition, sterile fixed oils may conventionally be employed as a solvent or suspending medium. For this purpose any bland fixed oil may be employed including synthetic mono- or diglycerides. In addition, fatty acids such as oleic acid may likewise be used in the preparation of injectables.
The amount of active ingredient, e.g. the compounds of formula I, which may be combined with the carrier material to produce a single dosage form will vary depending upon the host treated and the particular mode of administration. For example, a time-release formulation intended for oral administration to humans may contain approximately 20 to 500 mg of active material compounded with an appropriate and convenient amount of carrier material which may vary from about 5 to about 95% of the total compositions. It is preferred that the pharmaceutical composition be prepared which provides easily measurable amounts for administration. Typically, an effective amount to be administered systemically is about 0.1 mg/kg to about 100 mg/kg and depends upon a number of factors including, for example, the age and weight of the subject (e.g., a mammal such as a human), the precise condition requiring treatment and its severity, the route of administration, and will ultimately be at the discretion of the attendant physician or veterinarian. It will be understood, however, that the specific dose level for any particular patient will depend on a variety of factors including the activity of the specific compound employed, the age, body weight, general health, sex and diet of the individual being treated; the time and route of administration; the rate of excretion; other drugs which have previously been administered; and the severity of the particular condition undergoing therapy, as is well understood by those skilled in the art.

The present application also provides pharmaceutically acceptable salts, hydrates, solvates, isomers, tautomers, stereoisomers, enantiomers, racemates, atropisomers, polymorphs, prodrugs, or a mixture thereof, of the compounds described herein.

As used herein, pharmaceutically acceptable salts include, but are not limited, acetates, ascorbates, benzenesulphonates, benzoates, besylates, bicarbonates, bitartrates, bromides/hydrobromides, Ca-edetates/edetates, camsylates, carbonates, chlorides/hydrochlorides, citrates, edisylates, ethane disulphonates, estolates esylates, fumarates, gluceptates, gluconates, glutamates, glycolates, glycollylarsnilates, hexylresorcinates, hydrabamines, hydroxymaleates, hydroxynaphthoates, iodides, isothionates, lactates, lactobionates, malates, maleates, mandelates, methanesulphonates, mesylates, methylbromides, methylnitrates, methylsulphates, mucates, napsylates, nitrates, oxalates, pamoates, pantothenates, phenyl acetates, phosphates/diphosphates, polygalacturonates, propionates, salicylates, stearates, subacetates, succinates, sulphamides, sulphates, tannates, tartrates, teoclates, toluenesulphonates, triethiodides, ammonium, benzathines, chloroprocaines, cholines, diethanolamines, ethylenediamines, meglumines and procaines.
Further pharmaceutically acceptable salts can be formed with cations from metals like aluminium, calcium, lithium, magnesium, potassium, sodium, zinc and the like (also see Pharmaceutical salts, Birge, S.M. et al., J. Pharm. Sci., (1977), 66, 1- 19).

"Isomers" refers to compounds that have the same molecular formula. As used herein, the term isomers include double bond isomers, racemates, stereoisomers, enantiomers, diastereomers, and atropisomers. Single isomers, such as enantiomers or diastereomers, can be obtained by asymmetric synthesis or by resolution of a mixture of isomers. Resolution of a mixture of isomers (e.g. racemates) maybe accomplished, for example, by conventional methods such as crystallization in the presence of a resolving agent, or chromatography, using, for example a chiral high pressure liquid chromatography (HPLC) column. "Double bond isomers" refer to Z- and E- forms (or cis- and trans- forms) of the compounds with carbon-carbon double bonds.

"Racemates" refers to a mixture of enantiomers.

"Stereoisomers" or "stereoisomeric forms" refer to compounds that differ in the chirality of one or more stereocenters. Stereoisomers include enantiomers and diastereomers. The compounds may exist in stereoisomeric form if they possess one or more asymmetric centers or a double bond with asymmetric substitution and, therefore, can be produced as individual stereoisomers or as mixtures. Unless otherwise indicated, the description is intended to include individual stereoisomers as well as mixtures. The methods for the determination of stereochemistry and the separation of stereoisomers are well-known in the art (see, e.g., Chapter 4 of Advanced Organic Chemistry, 4th ed., J. March, John Wiley and * Sons, New York, 1992). [0019] "Tautomers" or "tautomeric formers" refer to alternate forms of a compound that differ in the position of a proton, such as enol-keto and imine-enamine tautomers, or heteroaryls such as pyrazoles, imidazoles, benzimidazoles, triazoles, and tetrazoles.

A "solvate" is formed by the interaction of a solvent and a compound. Solvates of salts of the compounds of any of the formulae described herein are also provided. Hydrates of the compounds of any of the formulae are also provided. "Hydrate" refers to a complex formed by the combining a compound disclosed herein and water. The term includes stoichiometric as well as non-stoichiometric hydrates.

A "prodrug" is defined in the pharmaceutical field as a biologically inactive derivative of a drug that upon administration to the human body is converted to the biologically active parent drug according to some chemical or enzymatic pathway.

A therapeutically effective amount for a particular patient may vary depending on factors such as the condition being treated, the overall health of the patient, the route and dose of administration and the severity of side effects. Guidance for methods of treatment and diagnosis is available (see, e.g., Maynard, et al. (1996) A Handbook of SOPs for Good Clinical Practice, Interpharm Press, Boca Raton, Fla.; Dent (2001) Good Laboratory and Good Clinical Practice, Urch Publ., London, UK).
An effective amount may be given in one dose, but is not restricted to one dose. Thus, the administration can be two, three, four, five, six, seven, eight, nine, ten, eleven, twelve, thirteen, fourteen, fifteen, sixteen, seventeen, eighteen, nineteen, twenty, or more, administrations of pharmaceutical composition. Where there is more than one administration of a pharmaceutical composition in the present methods, the administrations can be spaced by time intervals of one minute, two minutes, three, four, five, six, seven, eight, nine, ten, or more minutes, by intervals of about one hour, two hours, three, four, five, six, seven, eight, nine, ten, 11, 12, 13, 14, 15, 16, 17, 18, 19, 20, 21, 22, 23, 24 hours, and so on. In the context of hours, the term "about" means plus or minus any time interval within 30 minutes. The administrations can also be spaced by time intervals of one day, two days, three days, four days, five days, six days, seven days, eight days, nine days, ten days, 11 days, 12 days, 13 days, 14 days, 15 days, 16 days, 17 days, 18 days, 19 days, 20 days, 21 days, and combinations thereof. The invention is not limited to dosing intervals that are spaced equally in time, but encompass doses at non-equal intervals.

A dosing schedule of, for example, once/week, twice/week, three times/week, four times/week, five times/week, six times/week, seven times/week, once every two weeks, once every three weeks, once every four weeks, once every five weeks, and the like, is available for the invention. The dosing schedules encompass dosing for a total period of time of, for example, one week, two weeks, three weeks, four weeks, five weeks, six weeks, two months, three months, four months, five months, six months, seven months, eight months, nine months, ten months, eleven months, and twelve months.

Co- administration is also envisioned. Methods for co-administration with an additional therapeutic agent are well known in the art (Hardman, et al. (eds.) (2001) Goodman and Gilman's The Pharmacological Basis of Therapeutics, 10th ed., McGraw-Hill, New York, N.Y.; Poole and Peterson (eds.) (2001) Pharmacotherapeutics for Advanced Practice: A Practical Approach, Lippincott, Williams & Wilkins, Phila., Pa.; Chabner and Longo (eds.) (2001) Cancer Chemotherapy and Biotherapy, Lippincott, Williams & Wilkins, Phila., Pa.)

The present invention also concerns a method of treating and/or preventing a STING associated disease comprising administering a pharmaceutical composition of the invention.

Also envisioned is a method of blocking the activation-induced palmitoylation of STING and recruitment of TBK1 by contacting a cell expressing STING with a compound of the invention.

As disclosed above, the present invention also provides a kit. The terms "kit" and "pharmaceutical kit" refer to a commercial kit or package comprising, in one or more suitable containers, one or more pharmaceutical compositions of the invention, or compounds of the invention, and instructions for their use. Such kits may also be referred to by the terms "package" or "pharmaceutical package".

### EXAMPLES

### Methods

### Cells and cell culture conditions.

Cells were cultured under 5% CO₂ and 5% O₂ at 37•°C in Dulbecco's modified Eagle medium (DMEM) (Life Technologies) containing 10% (v/v) fetal bovine serum (FBS), 1% (v/v) penicillin (100•IU•ml^{•1})/streptomycin(100••g•ml^{•1}). Bone marrow-derived macrophages (BMDMs) were generated by culturing bone marrow cells from wild type mice in L929 conditioned medium (Sigma). MEFs were generated according to standard conditions. LL171 cells and their usage were described previously^{37 28}. WI-38 cells were purchased from ATCC CCL-75. THP-1 cells kindly provided by Angela Rösen-Wolff, University Hospital Dresden, Germany, and cultured in RPMI-1640 containing 10% (v/v) fetal bovine serum (FBS), 1% (v/v) penicillin (100•IU•ml^{•1})/streptomycin (100•• g•ml^{•1}).

### Mice and in vivo studies.

C57BL/6J mice (Stock#: 000664) were purchased from Jackson Laboratories. Trex1-deficient mice were a kind gift from Thomas Lindahl ³⁸ and were backcrossed for > 10 generations to C57BL/6NJ. All mice were maintained under specific pathogen-free (SPF) conditions at Ecole Polytechnique Fédérale de Lausanne (EPFL), Switzerland. For the pharmacokinetic studies, wild-type mice were injected intraperitoneally with 750 nmol C-176 per mouse in 200 • 1 corn oil (Sigma). Blood was collected at 30 min, 2 h and 4 h and serum C-176 levels were measured by mass spectrometry (LC-HRMS). To assess the *in vivo* inhibitory effect of C-176, wild-type mice (8-12 weeks of age) were injected either with vehicle or C-176. After 1 h or 4 h CMA was administered at a concentration of 224 mg kg⁻¹. 4 h later mice were sacrificed and the serum was collected to measure CMA-induced cytokine levels. The efficacy study in Trex1^{-/-} mice was conducted as follows: mice (2-5 weeks of age) were injected with 7,5 µl of C-176 (50 mM) or DMSO dissolved in 85 µl corn oil (Sigma) twice per day for 11 consecutive days. Mice were sacrificed by anaesthetization in a CO₂ chamber followed by cervical dislocation. All animal experiments were approved by the *Service de la consommation et des affaires vétérinaires* (1066 Épalinges, Canton of Vaud, Switzerland).

### High-throughput chemical compound screen.

HEK293T cells expressing murine STING with an N-terminal mCherry-tag ²⁸ were transfected using GeneJuice (Millipore) with a construct encoding for cyclic di-GMP synthase in conjunction with an IFN-• firefly luciferase reporter plasmid. 3 h after transfection cells were seeded in 348-well plates (Corning) coated with library compounds. For each compounds, a 40 nl volume was selected to obtain a concentration of 10 µM in the assay plates. The amount of DMSO in each well was normalised to 0.1%. After overnight treatment, cells were lysed in lysis buffer (25 mM Tris-phosphate (pH 7.8), 2 mM DTT, 2 mM 1,2-diaminocyclohexane-N,N,N',N'-tetraacetic acid, 10% glycerol, 1% Triton® X-100) for 20 min followed by the addition of firefly luciferase. Reporter activity was measured using a Tecan Infinite plate reader. The screen was performed using on ∼ 20.000 compounds from a chemically diverse compound collection available at the BSF core facility at EPFL.

### Histology.

Tissues were fixed in 4% formaldehyde solution (SAV LP), embedded in paraffin, and sections were stained with hematoxylin (Merck) and eosin (Shandon) (H&E). Four individual sections of the same heart were scored for levels of tissue damage (0-3) and inflammation (0-3). Scores represent the sum per heart.

### Type I IFN Bioassay and ELISA.

Mouse type I IFN levels were determined by incubating LL171 cells, stably expressing an ISRE-luciferase construct, with supernatants or serum for 5 h. LL171 cells were lysed in passive lysis buffer (Promega) and luciferase activity was measured using luciferin as substrate. Mouse IL-6 levels were determined by ELISA (BD Bioscience) according to the manufacturer's instructions.

### Stimulation of BMDMs.

BMDMs (1 × 10⁶ cells / ml) were pre-treated with DMSO or C-178 (0.25 µM unless indicated otherwise) for 1 h followed by stimulation with CMA (250 • g/ml; Sigma), dsDNA (90mer; 1.33 µg/mL), cyclic di-GMP or cyclic GAMP (1.5 µg /ml; Biolog Bremen). As control triphosphate RNA (160 ng) or LPS (1 µg/ ml; Invivogen) were used. To transfect dsDNA, triphosphate RNA and cyclic dinucleotides Lipofectamine 2000 (Life Technologies) was used. The sense strand of the 90mer DNA is as follows:

5[?]

TACAGATCTACTAGTGATCTATGACTGATCTGTACATGATCTACATACA

GATCTACTAGTGATCTATGACTGATCTGTACATGATCTACA-3'. (SEQ ID No. 1)

### RNA-Sequencing.

BMDMs were pre-treated with C-178 for 1 h followed by 2 h of stimulation with either DMSO or CMA. After CMA stimulation, RNA was isolated by RNeasy Mini kit (Qiagen). mRNA-seq libraries were prepared using the TruSeq mRNA stranded LT (Illumina kit). Samples were sequenced by the NextSeq 500 system sequencing with 1 × 75 cycle ('single read'), 'high output' mode. Resultant data files were converted to fastq format, demultiplexed into constituent libraries and trimmed. Short reads were aligned to the mouse genome mm10. Heatmaps of normalized values were generated using the web-based platform Automated Single-cell Analysis Pipeline³⁹.

### Western blot analysis.

Cells were lysed in 1 × Laemmli buffer or beads after immunoprecipitation were lysed in sample reducing buffer followed by denaturing at 95° C for 5 min. Cell lysates were separated by 12% SDS-PAGE and transferred onto PVDF membranes. Blots were incubated with anti-STING (D2P2F), Phospho-TBK1 (D52C2), TBK-1 (D1B4) (all Cell Signaling), anti-Transferrin Receptor (Thermo Scientific, 136800) or anti-Flag M2 (Sigma, F3165). As secondary antibodies, anti-rabbit-IgG-HRP or anti-mouse-IgG-HRP (1:2000 dilution) (Santa Cruz Biotechnology) were used. Anti-• -actin (C4, Santa Cruz, 1:5000 dilution) was used as control. ECL signal was recorded on the ChemiDoc XRS Biorad Imager and data were analysed with Image Lab (Biorad).

### Quantitative RT-qPCR.

Total RNA was isolated using the RNAeasy Mini Kit (Qiagen) and cDNA was synthesized using the RevertAid First Strand cDNA Synthesis kit (Fermentas). Quantitative RT-qPCR was performed in duplicates using Maxima SYBR Green Master Mix (Thermo Scientific) on a QuantStudio 5 machine. GAPDH was used as an endogenous normalization control to obtained relative expression data. Primer sequences are as follows:
mmGAPDH forward, 5•-GTCATCCCAGAGCTGAACG-3•; SEQ IDN No. 2
mmGAPDH reverse, 5•-TCATACTTGGCAGGTTTCTCC-3•; SEQ IDN No. 3
mmIFNB1 forward, 5•-CTCCAGCTCCAAGAAAGGAC-3•; SEQ IDN No. 4
mmIFNB1 reverse, 5•-TGGCAAAGGCAGTGTAACTC-3•; SEQ IDN No. 5
mmUSP18 forward, 5•-AAGGACCAGATCACGGACAC-3•; SEQ IDN No. 6
mmUSP18 reverse, 5•-CATCCTCCAGGGTTTTCAGA-3•-, SEQ IDN No. 7
mmISG15 forward, 5•-AAGAAGCAGATTGCCCAGAA-3•; SEQ IDN No. 8
mmISG15 reverse, 5•-TCTGCGTCAGAAAGACCTCA-3•; SEQ IDN No. 9
hsGAPDH forward, 5•-GAGTCAACGGATTTGGTCGT-3•; SEQ IDN No. 10
hsGAPDH reverse, 5•-GACAAGCTTCCCGTTCTCAG-3•; SEQ IDN No. 11
hsIFNB1 forward, 5•-CAGCATCTGCTGGTTGAAGA-3•; SEQ IDN No. 12
hsIFNB1 reverse, 5•-CATTACCTGAAGGCCAAGGA-3•SEQ IDN No. 13

### Metabolic labelling with [³H] palmitate.

Flag-STING expression was induced in HEK293T overnight by puromycin. Cells were starved for 1 h in IM (Glasgow minimal essential medium buffered with 10 mM Hepes, pH 7.4) with C-178 or C-176 (1 µM). Cells were then incubated for 2 h in IM with 200 µCi/ml ³H palmitic acid (9,10-³H(N)) (American Radiolabeled Chemicals, Inc.) in presence of C-178 or C-176 and with or without stimulation with CMA (250 • g/ml). For immunoprecipitation, cells were washed 3 times PBS, lysed with indicated antibodies (anti-FLAG and anti-hTfR) for 30 min at 4 °C in the following buffer (0.5% Nonidet P-40, 500 mM Tris pH 7.4, 20 mM EDTA, 10 mM NaF, 2 mM benzamidin and protease inhibitor cocktail (Roche)), and centrifuged 3 min at 5000 rpm. Supernatants were incubated overnight at 4°C with the appropriate antibodies and G Sepharose beads (GE Healthcare, 17-0618-01). For radiolabelling experiments, after immunoprecipitation, washed beads were incubated for 5 min at 90°C in reducing sample buffer prior to 4-20% gradient SDS-PAGE. Following SDS-PAGE gels were incubated in a fixative solution (25% isopropanol, 65% H₂O, 10% acetic acid) and incubated for 30 min with signal enhancer Amplify NAMP100 (GE Healthcare). The radiolabelled products were revealed using autoradiography and quantified using the Typhoon Imager (ImageQuanTool, GE Healthcare), the products were visulaised in parallel by immunoblotting with anti-FLAG or anti-hTfR antibodies.

### Competition Assay.

HEK293T cells expressing Flag-STING were seeded in 12-well plates and incubated in serum-free media with C-176-22 in combination with C-178 or C-176-09 at indicated concentrations for 30 min. Cells were harvested in PBS and analysed by in-gel analysis of C-176-22 labelling STING (see below).

### Immunoprecipitation.

Flag-STING expression was induced in HEK293T overnight by doxycycline. Cells were incubated with or without C-178 or C-176 [1µM] for 1 h and treated with DMSO or CMA (250 • g/ml) for 2 h. Cells were washed in PBS and lysed in lysis buffer (50 mM HEPES, 150 mM NaCl, 10% Glycerin, 1 mM MgCl, 1mM CaCl, 1% Brij-58 and protease inhibitor cocktail (Sigma P8340)) for 30min. Flag-STING was immunoprecipitated using anti-Flag M2 affinity gel agarose gel (Sigma) for 2 h at 4° C. After washing twice in lysis buffer and twice in PBS, the supernatant was completely removed and the resin was boiled in sample buffer before SDS-PAGE was performed.

### Blue Native Gel Assay.

Flag-STING expression was induced in HEK293T overnight by doxycycline. Cells were washed, collected and lysed using the native PAGE sample prep kit (Invitrogen) in 1% Digitonin according to manufacturer's instructions. Lysates were run on native PAGE 4-16% Bis-Tris gel and transferred on a PVDF membrane (Invitrogen).

### Gel-based analysis of C-176-22 labelling STING.

HEK293T cells expressing Flag-STING were incubated with C-176-22 (0.25 µM) in serum free media, collected in PBSs and lysed by repetitive freezing and thawing. 43 µl of lysed cells were treated with freshly prepared "click reagent" mixture containing tris(benzyltriazolylmethyl)amine (TBTA) (3 µl/sample, 3 mM in 1:4 DMSO:*t*-ButOH), tetramethylrhodamine (TAMRA) azide (2 µl/sample, 1.25 mM in DMSO), and freshly prepared CuSO₄ (1 µl/sample) and tris-(2-carboxyethyl)phosphine hydrochloride (TCEP) (1µl/sample) and incubated at room temperature for 1 h. The reaction was quenched by addition of reducing sample buffer. In-gel fluorescence was visualized using Fusion FX (Vilber Lourmat) and analysed by Fusion capt advance acquisition software.

### Lentiviral vector production and transduction.

HEK 293T cells were transfected with pCMVDR8.74, pMD2.G plasmids and the puromycin-selectable lentiviral vector pTRIPZ containing the open reading frame of Flag-mmSTING by the calcium phosphate precipitation method. The supernatant containing lentiviral particles was harvested at 48 and 72 h, pooled and concentrated by ultracentrifugation. WT MEFs and HEK293T cells were transduced with the lentiviral vectors by directly adding 5 • l of concentrated stock to the culture medium.

### Immunostaining.

MEFs were seeded on coverslips, fixed with 2% (v/v) paraformaldehyde for 10 min, permeabilised for 5 min in 0.1% (v/v) Triton X-100 and blocked with 2% BSA in PBS for 20 min at room temperature. Samples were incubated with the primary antibodies for 3 h (anti-flag M2 (F1804, sigma), Phospho-TBK1 (D52C2, Cell Signaling) and GM130 (clone 35, BD Pharmingen). After washing, samples were incubated with secondary antibodies (goat anti-rabbit Alexa Fluor 488 ot donkey anti-mouse Alexa Fluor 568 (Thermo Fisher)) containing 5 • g 6-diamino-2-phenylindole (DAPI) for 1 h at room temperature. Coverslips were mounted with Dak (fluorescent mounting medium). Images were acquired by a wide-field fluorescence microscope (Zeiss Axioplan) and processed in ImageJ software. Confocal sections were obtained with a confocal laser scanning microscope (Zeiss LSM710).

### Protein mass spectrometry.

Flag-STING or Flag-STING C91S expression was induced in HEK293T overnight by doxycycline. Cells were treated with or without C-178 or C-176 (1 µM) for 30min and lysed for in lysis buffer (20 mM Hepes, 150 mM NaCl, 10% glycerin and 1% DDM). Flag-STING was immunoprecipitated using anti-Flag M2 affinity gel agarose gel (Sigma) for 2 h at 4° C. Precipitated proteins were eluded using Flag peptide according to the manufacturer's instructions. Protein mass spectrometry was performed on a Shimadzu MS2020 connected to a Nexerra UHPLC system equipped with a Waters ACQUITY UPLC BEH C4 1.7µm 2.1x50mm column. Buffer A: 0.05% HCOOH in H₂O Buffer B: 0.05% HCOOH in acetonitrile. Analytical gradient was from 10% to 90% B within 6.0 min with 0.75 ml/min flow. MS was collected from 300-2000 Da and the spectra were deconvoluted using the software MagTran.

### Chemical synthesis.

All chemical reagents and anhydrous solvents for synthesis were purchased from commercial suppliers (Sigma-Aldrich, Fluka, Acros) and were used without further purification or distillation. The composition of mixed solvents is given by the volume ratio (v/v). 1H and 13C nuclear magnetic resonance (NMR) spectra were recorded on a Bruker DPX 400 (400 MHz for 1H, 100 MHz for 13C, respectively) or Bruker AVANCE III 400 Nanobay (400 MHz for 1H, 100 MHz for 13C, respectively) with chemical shifts (•) reported in ppm relative to the solvent residual signals of DMSO-d6 (2.50 ppm for 1H, 39.52 ppm for 13C). Coupling constants are reported in Hz. LC-MS was performed on a Shimadzu MS2020 connected to a Nexerra UHPLC system equipped with a Waters ACQUITY UPLC BEH C18 1.7µm 2.1x50mm column. Buffer A: 0.05% HCOOH in H2O Buffer B: 0.05% HCOOH in acetonitrile. Analytical gradient was from 10% to 90% B within 6.0 min with 0.5 ml/min flow.

### C-178

N-dibenzo[b,d]furan-3-yl-5-nitro-2-furamide. This compound has been purchased from ChemBridge (compound 5747493, www.hit2lead.com)

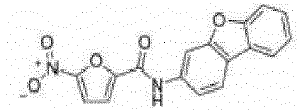

### C-176

N-(4-iodophenyl)-5-nitro-2-furamide. This compound has been purchased from Vitas-M Laboratory (compound STK016322, www.vitasmlab.com)

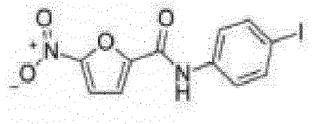

### C-176-002

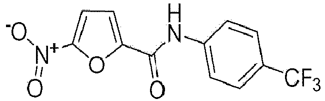

0.175 g of 5-nitro-2-furoyl chloride (1.0 eq., 1.00 mmoles) was dissolved in 4.000 mL of Dichloromethane. 0.1243 mL of 4-Aminobenzotrifluoride (1.0 eq., 1.00 mmoles) and 0.208 mL of Triethylamine (1.5 eq., 1.50 mmoles) were added. The reaction was stirred for 4h at r.t. The reaction mixture was poured in a well stirred mixture of DCM (ml) and 10% citric acid (ml). The phases were separated and the organic phase was extracted with sat. NaHCO3 (25 ml). organic phase was dried over MgSO4, filtered and evaporated under vacuum. The residue was dissolved in a minimum of AcOEt and an equal volume of Hexane was added which resulted in the formation of a yellow crystalline solid. The solid was filtered, washed with AcOEt/Hx 1:2 and dried under high vacuum. Yield: 82 mg (27%). ¹H NMR (400 MHz, DMSO) • 10.96 (s, 1 H), 7.99 (d, 2 H, *J* = 8.5 Hz), 7.84 (d, 1 H, *J* = 3.9 Hz), 7.78 (d, 2 H, *J* = 8.6 Hz), 7.70 (d, 1 H, *J* = 3.9 Hz). LC: Rt = 3.52 min.

### C-176-009

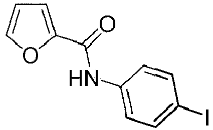

0.130 g of 2-furoyl chloride (1.0 eq., 1.00 mmoles) was dissloved in 2.0 mL of DMF. Separately 0.235 g of 4-iodoaniline (1.08 eq., 1.08 mmoles) and 0.208 mL of Triethylamine (1.5 eq., 1.50 mmoles) were dissolved in 2.0 ml DMF, the mixture was added to the acyl chloride solution and the reaction was stirred at r.t. for 2 h. The reaction mixture was poured in 10% citric acid (25 ml) which resulted in the formation of a solid. The solid was filtered, washed with 10% citric acid (10 ml) Sat. NaHCO3 (10 ml), Water (10 ml) and Methanol (1 ml). The greyish solid was then and dried under high vacuum. Yield: 108 mg (34%). ¹H NMR (400 MHz, DMSO) • 10.29 (s, 1 H), 7.96 (m, 1 H), 7.68 (m, 2 H), 7.61 (m, 2 H), 7.35 (m, 1 H), 6.72 (dd, 1 H, *J* = 1.7, 3.5 Hz). LC: Rt = 3.51 min.

### C-176-18

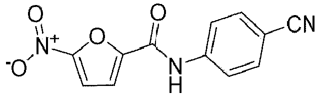

0.157 g of 5-notro-2-furoyl chloride (1.0 eq., 1.00 mmoles) was dissolved in 2.0 mL of DMF. Separately 0.126 g of 4-iodoaniline (1.08 eq., 1.08 mmoles) and 0.208 mL of triethylamine (1.5 eq., 1.50 mmoles) were dissolved in 2.0 mL DMF. The resulting mixture was added to the acyl chloride solution and the reaction was stirred at room temperature for 2 h. The reaction mixture was poured in 10% citric acid (25 mL) which resulted in the formation of a solid. The solid was filtered, washed with 10% citric acid (10 mL), a saturated solution of NaHCO₃ (10 mL), water (10 mL) and methanol (1 mL). The light green solid was then and dried under high vacuum to afford *N*-(4-cyanophenyl)-5-nitrofuran-2-carboxamide. Yield: 157 mg (61 %). ¹H NMR (400 MHz, DMSO) • 11.01 (s, 1 H), 7.96 (m, 2 H, *J* = 8.8 Hz), 7.87 (m, 2 H, *J* = 8.8 Hz), 7.85 (d, 1 H, *J* = 3.9 Hz), 7.70 (d, 1 H, *J* = 3.9 Hz). ¹³C NMR (101 MHz, DMSO) • 155.5, 152.4, 147.7, 142.7, 133.8, 121.1, 119.4, 117.9, 113.9, 106.8. LC: Rt = 4.02 min. HRMS (ESI) calcd for C₁₂H₆N₃O₄ [M-H]- 256.0358; found 256.0362.

### C-176-021

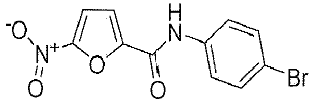

0.088 g of 5-nitro-2-furoyl chloride (1.0 eq., 0.50 mmoles) was dissloved in 1.0 mL of DMF. Separately 0.092 g of 4-bromoaniline (1.08 eq., 0.54 mmoles) and 0.104 mL of triethylamine (1.5 eq., 0.75 mmoles) were dissolved in 1.0 ml DMF, the solution was added to the acyl chloride solution and the reaction was stirred at r.t. for 2 h. The reaction mixture was poured in 10% citric acid (25 ml) which resulted in the formation of a yellow solid. The solid was filtered, washed with 10% citric acid (10 ml) Sat. NaHCO3 (10 ml), Water (10 ml) and Methanol (1 ml). The yellow solid was then and dried under high vacuum. Yield: 92 mg (35%). ¹H NMR (400 MHz, DMSO) • 10.76 (s, 1 H), 7.83 (d, 1 H, *J* = 3.8 Hz), 7.73 (m, 2 H, *J* = 8.7 Hz), 7.65 (d, 1 H, *J* = 3.8 Hz), 7.59 (d, 2 H, *J* = 8.7 Hz). LC: Rt = 3.36 min.

### C-176-22

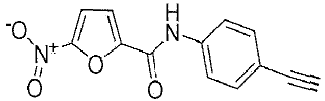

0.088 g of 5-nitro-2-furoyl chloride (1.0 eq., 0.50 mmoles) was dissloved in 1.0 mL of DMF. Separately 0.063 g of 4-ethynylaniline (1.08 eq., 0.54 mmoles) and 0.104 mL of triethylamine (1.5 eq., 0.75 mmoles) were dissolved in 1.0 ml DMF, the solution was added to the acyl chloride solution and the reaction was stirred at r.t. for 2 h. The reaction mixture was poured in 10% citric acid (25 ml) which resulted in the formation of a yellow solid. The solid was filtered, washed with 10% citric acid (10 ml) Sat. NaHCO3 (10 ml), Water (10 ml) and Methanol (1 ml). The orange solid was then and dried under high vacuum. Yield: 91 mg (71%). ¹H NMR (400 MHz, DMSO) • 10.79 (s, 1 H), 7.83 (d, 1 H, *J* = 3.9 Hz), 7.78 (d, 2 H, *J* = 8.6 Hz), 7.66 (d, 1 H, *J* = 3.9 Hz), 7.51 (d, 2 H, *J* = 8.5 Hz), 4.17 (s, 1 H). LC: Rt = 3.38 min.

### C-176-025

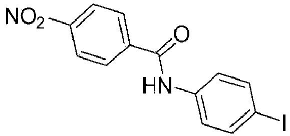

0.167 g of 4-nitrobenzoic acid (1.0 eq., 1.00 mmoles), 0.219 g of 4-Iodoaniline (1.0 eq., 1.00 mmoles), 0.305 g of 4-Dimethylaminopyridine (2.5 eq., 2.50 mmoles) and 0.288 g of 1-(3-Dimethylaminopropyl)-3-ethylcarbodiimide hydrochloride (1.5 eq., 1.50 mmoles) were dissolved in 2.6 mL of N,N-Dimethylformamide. The reaction was stirred 2h at r.t., diluted with 75 ml AcOEt, washed with 0.1M HCl (2 x 50 ml), washed with sat. NaHCO₃ (2 x 50 ml),dried over MgSO4, filtered and evaporated under vacuum. The solid residue was triturated in AcOEt/hexane 1:1 (10ml), filtered, washed with 1ml AcOEt/hexane 1:1 and dried under high vacuum. Yield: 248 mg (67%). ¹H NMR (400 MHz, DMSO) • 10.67 (s, 1 H), 8.38 (d, 2 H, *J* = 8.8 Hz), 8.18 (d, 2 H, *J* = 8.8 Hz), 7.73 (m, 2 H, *J* = 8.7 Hz), 7.64 (m, 2 H, *J* = 8.7 Hz). LC: Rt = 3.99 min.

### Statistical analysis.

All data are plotted as mean values with variance as s.e.m. unless stated otherwise. Statistical analysis was performed using Prims 6 (Graphpad Software Inc.). Normal distribution was assumed for all quantitative measurements. No statistical methods were used to predetermine sample sizes.

### Results

### A high-throughput chemical screen identifies two small molecule inhibitors of the STING signalling pathway

To identify small molecules that specifically inhibit STING, we established a cell-based screening assay based on previously described HEK293T cells stably expressing a mouse, mCherry-tagged STING construct (Fig. 6a)²⁸. In these cells, transient transfection of vectors encoding for cyclic di-GMP synthase (cdG-Syn) and an IFN-• reporter leads to robust induction of firefly luciferase activity. We used this system to perform a high-throughput chemical screen with a library of ∼ 20.000 small molecule compounds (Fig. 6b). To increase the selectivity of candidate compounds against the STING pathway, hits from the primary screen were counter-screened for their effect on RIG-I-driven activation of the IFN-• reporter. Amongst the compounds that selectively reduced the IFN signal in response to cdG-Syn/STING but not to RIG-I, we identified C-178 and C-176, both containing a similar chemical scaffold with a nitrofuran group (Fig. 1a and Fig. 6c-f). To further validate the two small molecules, we treated wild-type HEK293T cells with C-178 and C-176, in which STING was either expressed alone or in conjunction with cdG-Syn. Strikingly, both compounds caused dose-dependent reduction of IFN-• reporter activity with nanomolar half-maximal inhibitory concentration (IC₅₀) values, whereas neither RIG-I nor TBK1-driven IFN induction was affected (Fig. 1b). Similar inhibitory effects on STING signalling were observed in HEK293T cells expressing STING together with cGAS or cyclic di-AMP synthase (Data not shown) indicating that C-178 and C-176 antagonize STING in a ligand-independent manner. We next conducted a limited structure-activity relationship (SAR) analysis. Since both C-178 and C-176 share a characteristic nitrofuran moiety, we prepared analogues of C-176/8 in which this structural motif was substituted (Fig. 1c). Interestingly, derivatives with substituents of either the nitro group or the furan group completely lost their inhibitory capacity against STING (Fig. 1d). We also modified the side chains of the aromatic ring (Fig. 1c), Whereas these analogues maintained a similar inhibitory effect on STING, some also reduced the RIG-I signal (Fig. 1d), Therefore, the inhibitory activity of C-178 and C-176 appears to rely on the presence of the reactive nitrofuran scaffold, while substituents of the aromatic ring can fine-tune the inhibitory potency but influence off-target effects at the same time. Given the higher potency of C-178 compared to C-176 with regards to IC₅₀ values, we prioritized that compound for the following studies.

### C-178 is active against STING in primary cells

We next tested the effect of C-178 on STING-dependent activation in mouse bone marrow-derived macrophages (BMDMs). Pre-treatment of BMDMs with C-178 followed by stimulation with cGAMP dose-dependently inhibited the production of type I IFNs with a calculated IC₅₀ of approximately 23 nM (Fig. 2a). Likewise, cGAMP-induced expression of IFN-• mRNA was inhibited by C-178 (Fig. 2b). In contrast, C-178 did not affect the production of type I IFNs from BMDMs stimulated with triphosphate RNA, an activator of the RIG-I pathway (Fig. 2a). Similar to the effect observed for cGAMP, C-178 reduced type I IFN responses triggered by other *bona fide* STING activators including cyclic di-GMP, the small molecule ligand CMA and 90bp dsDNA, but it was not active against the TLR-4 agonist LPS as an additional control (Fig. 2c). To systematically profile the effect of C-178 on the global transcriptional program initiated by STING, we performed RNA sequencing in BMDMs stimulated with CMA in the presence or absence of C-178 (Fig. 2d). Of note, exposure to C-178 alone did not majorly affect the gene expression profile of BMDMs relative to DMSO-treated samples (30 genes up-regulated, 9 genes down-regulated, cut-off log₂ fold change >1, *P* < 0.05). As expected, stimulation with CMA resulted in profound changes in gene expression (1467 up-regulated, 829 down-regulated, cut-off log₂ fold change >1, *P* < 0.05). Remarkably, pre-treatment with C-178 significantly affected the induced expression of 99.6% (498) of the 500 most up-regulated genes after treatment with CMA, including well-known IRF-3 as well as NF-• B target genes. Thus, C-178 appears to influence various branches of the STING signaling cascade. Following its activation, STING recruits and activates TBK1, a step which is considered critical for the regulation of STING-dependent transcriptional responses ^{16,29}. We found that C-178 dose-dependently inhibited the phosphorylation of TBK1 induced by CMA, while not affecting the activation of TBK1 by triphosphate RNA or LPS (Fig. 2e, f). These results suggest that the mechanism by which C-178 inhibits the STING response in BMDMs targets an upstream step in the underlying signal transduction cascade.
In addition to BMDMs, the STING-triggered induction of type I IFNs was potently inhibited by C-178 in mouse embryonic fibroblasts (MEFs) (Fig. 2g). However, C-178 did not substantially alter the cGAMP response of human cells, including THP-1 cells and WI-38 cells, even at high concentrations (Fig. 2h, i). Analysing the IFN response of HEK293T cells overexpressing STING supported the conclusion that C-178 selectively acts on STING in mouse cells (see Fig. 3a). The observation of a seemingly species-specific activity of C-178 is of major significance. In fact, it suggests that C-178 directly acts on STING rather than targeting a signalling co-factor or disturbing the intactness of a more general cellular process necessary for the functionality of the STING pathway (e.g., ER-to-Golgi translocation). Since a direct antagonist of STING has not been reported to date, we thus went on to further understand the mechanism of action of C-178.

### C-178 targets the transmembrane region of STING

Previous studies have described two mouse-specific small molecule activators of STING, DMXAA and CMA, acting through non-conserved amino acid residues located within the ligand-binding domain (LBD) of murine STING ^{11,30 9}. To study whether the LBD, or perhaps other parts of STING, confer responsiveness to C-178, we generated chimeric STING expression constructs, in which the N-terminal domain (containing the LBD domain) and the C-terminal domain (including the transmembrane (TM) segments) of murine STING were fused to the C- and N-terminal part of human STING, respectively. To our surprise, C-178 blocked the chimeric construct that contained the N-terminal part of mouse STING fused to the C-terminal part of human STING, whereas C-178 was inactive against the corresponding reversed construct (Fig. 3a). These data therefore indicate that C-178 achieves inhibition of STING by targeting the poorly characterised N-terminal portion encompassing the transmembrane domains. Intrigued by these unexpected results, we next sought to more precisely map amino acids involved in the inhibitory activity of C-178. To this end, we individually mutated 108 amino acids (alanine-scanning) located in the N-terminal part of murine STING (AA21-AA138) and screened for mutants with compromised responsiveness to C-178. In order to reliably assess the effect of C-178, we required that an individual mutant maintained at least 25% of the activity of wild-type STING in the absence of inhibition. On the basis of these criteria, we identified two mutants, V27A and C91A, that exhibited severely compromised inhibition by C-178 (Fig. 3b). Interestingly, both mutants mapped to predicted TM domains of STING - TM1 for V27 and TM3 for C91A, respectively. Taken together, these data imply that C-178 antagonises STING by an entirely unexpected mechanism involving its TM region rather than the LBD.

### Irreversible inhibition of STING by C-178 through covalent binding to cysteine 91

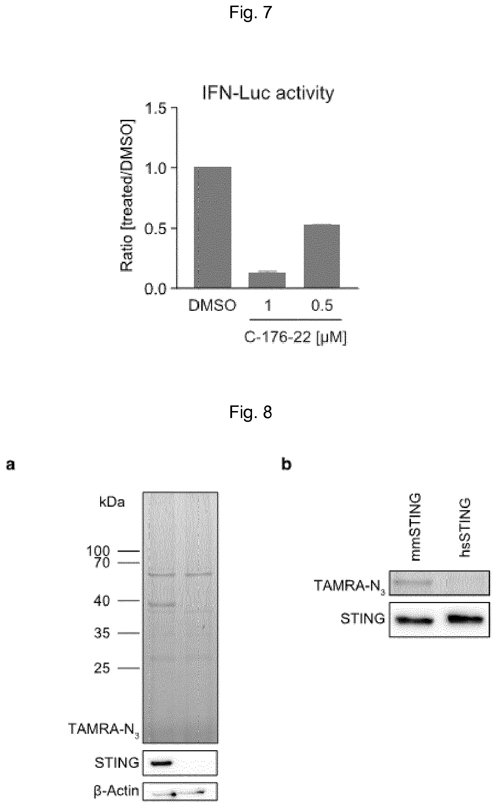

We next sought to elucidate the molecular details of the interaction between C-178 and the TM part of STING. Our SAR studies highlighted the importance of the nitrofuran motif for the activity of the discovered inhibitors. Given the strong electrophilic potential of this group, we suspected that C-178 might react with a TM residue generating a covalent interaction. In support of an irreversible mechanism of inhibition, washing BMDMs prior to stimulation with cGAMP did not reduce the inhibitory effect of C-178 (Fig. 3c). To further test the hypothesis of covalent engagement of STING, we synthesized a derivative of C-176-22, which bears an alkyne group amenable for copper-catalyzed azide alkyne cycloaddition (Click Chemistry) and performed gel-based profiling studies (Fig. 3d). Having verified that C-176-22 is still active (Fig. 7), HEK293T cells expressing STING were exposed to C-176-22, lysed, coupled to a tetramethylrhodamine (TAMRA-)azide tag and C-176-22-modified proteins were visualized using in-gel fluorescence scanning. HEK293T cells without STING served as a control for assessing non-specific protein labelling. Notably, treating cells with C-176-22 produced a marked, dose-dependent signal corresponding to a target protein exactly matching the size of STING (37 kDa) (Fig. 3f). In contrast, this band was not detectable in HEK293T cells that lacked STING expression and hardly detectable, if at all, in HEK293T cells that expressed human STING (Fig. 8a, b). Apart from STING, we observed negligible labelling of other protein targets, thus indicating that C-178 binds to STING with high proteome-wide selectivity. Gel-based competition studies showed that C-178, but not an inactive control molecule, dose-dependently blocked labelling of STING by C-176-22 (Fig. 3e). In addition, mass spectrometry confirmed that C-178 and as an additional control C-176 irreversibly modify STING (Fig. 3g and Fig. 9). Based on the observed mass shift, we postulate a nitrobenzyl rearrangement-like reaction to generate covalent bonding between C-178 and STING (see below). Together, these data provide strong evidence that C-178 acts as a selective, covalent inhibitor of STING.
We next sought to identify the residue through which C-178 achieves binding to STING. Among the naturally occurring amino acids, cysteine residues are well-known targets of electrophilic small molecules. Our results from the alanine-scanning revealed an essential role of cysteine 91 for the inhibitory activity of C-178. Indeed, we found that introducing a cysteine-to-alanine or cysteine-to-serine mutation at position 91 completely abolished the labelling of STING by C-176-22 (Fig. 3h). As a control, mutating C88 did not influence the interaction of STING with C-176-22. We confirmed the absence of covalent binding of C-178 and C-176 to STING-C91S by mass spectrometry (Fig. 9a, b). Interestingly, however, C-176-22 efficiently interacted with the non-responsive mutant STING V27A. Together, these data demonstrate that C-178 acts on STING by forming a covalent bond with cysteine 91 (Fig. 11). In addition, binding to and inhibition of STING are two separable functions of C-178.

### C-178 blocks activation-induced palmitoylation of STING and recruitment of TBK1

We next asked how the modification introduced by C-178 to the TM region of STING could block its downstream signalling capability. This is relevant since previous studies reported that the LBD of STING alone was sufficient to recruit TBK1 *in vitro* ²⁹. To address this question, we first examined whether C-178 might affect the intracellular trafficking process of STING. For this purpose, the activation-dependent translocation of STING from the ER to the Golgi was monitored in MEFs expressing recombinant FLAG-tagged STING. As expected, stimulation with CMA triggered the re-localization of STING towards the Golgi compartment (co-staining with GM310) and the concurrent phosphorylation of TBK-1 (Fig. 4a). Interestingly, pre-treatment of MEFs with C-178 did not affect the translocation of STING, despite completely abolishing the phosphorylation of TBK1 (Fig. 4a). After passing the Golgi, STING was shown to undergo degradation in autophagosomes ¹⁹. Kinetic studies revealed that C-178 did not impair CMA-triggered degradation of STING (Fig. 4b). This shows that C-178 does not prevent signalling by interfering with the trafficking of STING. We next considered that C-178 may inhibit the signalling function of STING by altering its posttranslational modifications. Amongst the different types of posttranslational modifications occurring on STING, palmitoylation caught our interest since it was reported to occur at cysteine 91 the targeted residue of C-178 ³¹. To test whether C-178 affects palmitoylation of STING, we utilized a [³H] palmitate labelling approach in HEK29T cells stably expressing STING. In agreement with previous results, we found that activation of STING by CMA induced pronounced palmitoylation as indicated by the strong increase in [³H] signal (Fig. 4c). Remarkably, [³H] palmitate labelling was severely reduced in cell pre-treated with C-178. As controls, C-176 also inhibited [³H] palmitate labelling of STING, but neither C-178 nor C-176 affected the labelling of the endogenous transferrin receptor (Fig. 15a, b). Palmitoylation has been described to facilitate the multimerisation of modified proteins ³². In the context of STING, the formation of a multimeric complex is proposed to be an essential step for the recruitment of TBK1 ¹⁶. Thus, we next investigated whether C-178 affects clustering of STING and/or the recruitment of native TBK1. Following activation by CMA, we observed the formation of high molecular weight complexes by STING, which paralleled with the recruitment of TBK1 (Fig. 4d, e). Of note, treating cells with C-178, C-176 or 2-bromopalmitate, a non-selective inhibitor of palmitoylation, blocked the assembly of STING clusters (Fig. 4d and Fig. 12c). Moreover, the absence of cluster formation upon C-178 treatment concurred with an impaired recruitment of TBK1 (Fig. 4e and Fig. 12d). Together, this demonstrates that C-178 interferes with the initiation of downstream signalling by blocking palmitoylation and preventing the recruitment of TBK1.

### C-176 inhibits STING in vivo and attenuates the pathology of autoinflammatory disease

We next studied the effects of pharmacological inhibition of STING *in vivo.* Since we noticed improved solubility of C-176 relative to C-178, we chose this compound for the *in vivo* studies. First, to determine the bioavailability and half-life of C-176 we performed a single-dose pharmacokinetic study. Following intraperitoneal (i.p.) injection C-176 peaked in the serum after 30 min and then rapidly decreased (Fig. 13). We next evaluated whether the systemic induction of type I IFNs triggered by the administration of CMA could be blocked by C-176. Mice were pre-treated with C-176 for 1 h or 4 h before i.p. injection of CMA and 4 h later the production of type I IFNs and IL-6 in the serum was assessed. Remarkably, C-176 completely abrogated the increase in serum concentration of both type I IFNs and IL-6 (Fig. 5a). Moreover, its potency was not affected by the different time interval in between the administration of C-176 and the injection of CMA (Fig. 5a). These data demonstrate that C-176 is effective *in vivo* and suggest that its *in vivo* inhibitory capacity is not limited by the short serum half-life, as expected for a covalent inhibitor. Finally, we moved on to test whether C-176 were effective in models of STING-mediated autoinflammatory disease. Deficiency for the DNase TREX1 has been shown to trigger severe systemic inflammation in mice affecting several organs, most mentionable the heart, which is caused by a pathological cytokine signature (type I IFNs) due to persistent activation of the cGAS-STING pathway ^{23,33-35}. We first tested whether pharmacological inhibition of STING could reduce the spontaneous ISG expression evident in MEFs from TREX1 KO mice. Whereas TREX1 KO MEFs displayed an elevated ISG signature (ISG15, USP18, IP10), treatment with C-178 markedly reduced ISGs levels (Fig. 5b). We also noted that, C-178 blocked the responsiveness of cells to a mutant version of STING (STINGV154M), which corresponds to a gain-of-function mutation found in SAVI patients ^{21,22} (Fig. 14). This indicated that small molecule-mediated inhibition of STING could indeed be effective in STING-driven autoinflammation. We then performed a two-week efficacy study in TREX1 KO mice, in which C-176 was daily delivered to diseased mice shortly after birth. Treatment of mice with C-176 resulted in a significant decrease in the serum concentration of type I IFNs compared to mice that received vehicle only. Histological analysis of the heart after treatment showed decreased cellularity consistent with reduced inflammation (Fig. 5c-e). Of importance, there were no signs of overt toxicity (weight changes) compared with mice treated with vehicle alone. In sum, these data show that C-176 potently blocks STING *in vivo,* whether induced by a synthetic STING agonist or in the setting of endogenous STING-driven autoinflammation.

### REFERENCES

1 Ishikawa, H., Ma, Z. & Barber, G. N. STING regulates intracellular DNA-mediated, type I interferon-dependent innate immunity. Nature 461, 788-792, doi:10.1038/nature08476 (2009).
2 Ishikawa, H. & Barber, G. N. STING is an endoplasmic reticulum adaptor that facilitates innate immune signalling. Nature 455, 674-678, doi:10.1038/nature07317 (2008).
3 Sun, L., Wu, J., Du, F., Chen, X. & Chen, Z. J. Cyclic GMP-AMP synthase is a cytosolic DNA sensor that activates the type I interferon pathway. Science 339, 786-791, doi:10.1126/science.1232458 (2013).
4 Wu, J. et al. Cyclic GMP-AMP is an endogenous second messenger in innate immune signaling by cytosolic DNA. Science 339, 826-830, doi:10.1126/science.1229963 (2013).
5 Gao, P. et al. Cyclic [G(2',5')pA(3',5')p] is the metazoan second messenger produced by DNA-activated cyclic GMP-AMP synthase. Cell 153, 1094-1107, doi:10.1016/j.cell.2013.04.046 (2013).
6 Zhang, X. et al. Cyclic GMP-AMP containing mixed phosphodiester linkages is an endogenous high-affinity ligand for STING. Molecular cell 51, 226-235, doi:10.1016/j.molcel.2013.05.022 (2013).
7 Diner, E. J. et al. The innate immune DNA sensor cGAS produces a noncanonical cyclic dinucleotide that activates human STING. Cell reports 3, 1355-1361, doi:10.1016/j.celrep.2013.05.009 (2013).
8 Burdette, D. L. et al. STING is a direct innate immune sensor of cyclic di-GMP. Nature 478, 515-518, doi:10.1038/nature10429 (2011).
9 Cavlar, T., Deimling, T., Ablasser, A., Hopfner, K. P. & Hornung, V. Species-specific detection of the antiviral small-molecule compound CMA by STING. The EMBO journal 32, 1440-1450, doi:10.1038/emboj.2013.86 (2013).
10 Conlon, J. et al. Mouse, but not human STING, binds and signals in response to the vascular disrupting agent 5,6-dimethylxanthenone-4-acetic acid. Journal of immunology 190, 5216-5225, doi:10.4049/jimmunol.1300097 (2013).
11 Huang, Y. H., Liu, X. Y., Du, X. X., Jiang, Z. F. & Su, X. D. The structural basis for the sensing and binding of cyclic di-GMP by STING. Nature structural & molecular biology 19, 728-730, doi:10.1038/nsmb.2333 (2012).
12 Shang, G. et al. Crystal structures of STING protein reveal basis for recognition of cyclic di-GMP. Nature structural & molecular biology 19, 725-727, doi:10.1038/nsmb.2332 (2012).
13 Shu, C., Yi, G., Watts, T., Kao, C. C. & Li, P. Structure of STING bound to cyclic di-GMP reveals the mechanism of cyclic dinucleotide recognition by the immune system. Nature structural & molecular biology 19, 722-724, doi:10.1038/nsmb.2331 (2012).
14 Yin, Q. et al. Cyclic di-GMP sensing via the innate immune signaling protein STING. Molecular cell 46, 735-745, doi:10.1016/j.molce1.2012.05.029 (2012).
15 Dobbs, N. et al. STING Activation by Translocation from the ER Is Associated with Infection and Autoinflammatory Disease. Cell host & microbe 18, 157-168, doi:10.1016/j.chom.2015.07.001 (2015).
16 Liu, S. et al. Phosphorylation of innate immune adaptor proteins MAVS, STING, and TRIF induces IRF3 activation. Science 347, aaa2630, doi:10.1126/science.aaa2630 (2015).
17 McWhirter, S. M. et al. A host type I interferon response is induced by cytosolic sensing of the bacterial second messenger cyclic-di-GMP. The Journal of experimental medicine 206, 1899-1911, doi:10.1084/jem.20082874 (2009).
18 Abe, T. & Barber, G. N. Cytosolic-DNA-mediated, STING-dependent proinflammatory gene induction necessitates canonical NF-kappaB activation through TBK1. Journal of virology 88, 5328-5341, doi:10.1128/JVI.00037-14 (2014).
19 Konno, H., Konno, K. & Barber, G. N. Cyclic dinucleotides trigger ULK1 (ATG1) phosphorylation of STING to prevent sustained innate immune signaling. Cell 155, 688-698, doi:10.1016/j.cell.2013.09.049 (2013).
20 Crow, Y. J. & Manel, N. Aicardi-Goutieres syndrome and the type I interferonopathies. Nature reviews. Immunology 15, 429-440, doi:10.1038/nri3850 (2015).
21 Liu, Y. et al. Activated STING in a vascular and pulmonary syndrome. The New England journal of medicine 371, 507-518, doi:10.1056/NEJMoa1312625 (2014).
22 Jeremiah, N. et al. Inherited STING-activating mutation underlies a familial inflammatory syndrome with lupus-like manifestations. The Journal of clinical investigation 124, 5516-5520, doi:10.1172/JCI79100 (2014).
23 Gall, A. et al. Autoimmunity initiates in nonhematopoietic cells and progresses via lymphocytes in an interferon-dependent autoimmune disease. Immunity 36, 120-131, doi:10.1016/j.immuni.2011.11.018 (2012).
24 Konig, N. et al. Familial chilblain lupus due to a gain-of-function mutation in STING. Annals of the rheumatic diseases 76, 468-472, doi:10.1136/annrheumdis-2016-209841 (2017).
25 Yan, N. Immune Diseases Associated with TREX1 and STING Dysfunction. Journal of interferon & cytokine research : the official journal of the International Society for Interferon and Cytokine Research 37, 198-206, doi:10.1089/jir.2016.0086 (2017).
26 Ahn, J., Gutman, D., Saijo, S. & Barber, G. N. STING manifests self DNA-dependent inflammatory disease. Proceedings of the National Academy of Sciences of the United States of America 109, 19386-19391, doi:10.1073/pnas.1215006109 (2012).
27 Ahn, J. et al. Inflammation-driven carcinogenesis is mediated through STING. Nature communications 5, 5166, doi:10.1038/ncomms6166 (2014).
28 Ablasser, A. et al. Cell intrinsic immunity spreads to bystander cells via the intercellular transfer of cGAMP. Nature 503, 530-534, doi:10.1038/nature12640 (2013).
29 Tanaka, Y. & Chen, Z. J. STING specifies IRF3 phosphorylation by TBK1 in the cytosolic DNA signaling pathway. Science signaling 5, ra20, doi:10.1126/scisignal.2002521 (2012).
30 Gao, P. et al. Structure-function analysis of STING activation by c[G(2',5')pA(3',5')p] and targeting by antiviral DMXAA. Cell 154, 748-762, doi:10.1016/j.cell.2013.07.023 (2013).
31 Mukai, K. et al. Activation of STING requires palmitoylation at the Golgi. Nature communications 7, 11932, doi:10.1038/ncomms11932 (2016).
32 Blaskovic, S., Blanc, M. & van der Goot, F. G. What does S-palmitoylation do to membrane proteins? The FEBS journal 280, 2766-2774, doi:10.1111/febs.12263 (2013).
33 Stetson, D. B., Ko, J. S., Heidmann, T. & Medzhitov, R. Trex1 prevents cell-intrinsic initiation of autoimmunity. Cell 134, 587-598, doi:10.1016/j.cell.2008.06.032 (2008).
34 Gray, E. E., Treuting, P. M., Woodward, J. J. & Stetson, D. B. Cutting Edge: cGAS Is Required for Lethal Autoimmune Disease in the Trex1-Deficient Mouse Model of Aicardi-Goutieres Syndrome. Journal of immunology 195, 1939-1943, doi:10.4049/jimmunol.1500969 (2015).
35 Gao, D. et al. Activation of cyclic GMP-AMP synthase by self-DNA causes autoimmune diseases. Proceedings of the National Academy of Sciences of the United States of America 112, E5699-5705, doi:10.1073/pnas.1516465112 (2015).
36 Crowl, J. T., Gray, E. E., Pestal, K., Volkman, H. E. & Stetson, D. B. Intracellular Nucleic Acid Detection in Autoimmunity. Annual review of immunology 35, 313-336, doi:10.1146/annurev-immunol-051116-052331 (2017).

## Claims

1. A compound of formula (I): 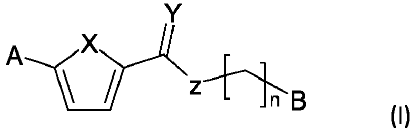 racemates, enantiomers, diastereomers and pharmaceutically acceptable salts thereof, wherein
A is selected from the group consisting of -NO₂, -C(O)OH, -C(O)O-, -C(O)OR¹,-C(O)R¹, -C(O)NH₂, -C(O)NHR¹, -C(O)NR²R³, -S(O)R¹, -SO₂R¹, -S(O)₂NH₂, -S(O)₂NHR¹,-S(O)₂NR²R^{3,} -S(O)(NR¹)R², -P(O)(OH)₂, -P(O)OHR¹, and -P(O)OR¹R²;
R¹, R² and R³ are each independently selected from H, halogen, C₁-C₆ alkyl, C₂-C₆ alkenyl, C₁-C₆ haloalkyl, C₃-C₇ cycloalkyl, -OH, -OR₄, -SH, -SR⁴, -NH₂, -NHR⁴ and -N(R⁴)₂, wherein R⁴ is C₁-C₆ alkyl optionally substituted with halogen, -OH, -CN, -NH₂ or =O;
or R² and R³ together with the nitrogen atom to which they are attached form a substituted or unsubstituted 4-7 membered heterocycle;
X and Z are each independently selected from O, S, or N;
Y is O or S;
B is selected from the group consisting of 6-31 membered aryl; 6-31 membered heteroaryl, 5-31 membered aryloxy, 5-31 membered aralkyl, optionally substituted with 1, 2, 3 substituents independently selected from -O-CF₃, -CF₃, -O-CHF₂, halogen, -OH, -NH₂,-CN, -OCH₃, C₁-C₆ alkyl, allyl, -C(O)OR¹, -C(O)NH₂, propargyl, -NO₂, -S(O)₂NH₂, and-S(O)CH₃; and
n is an integer between 0-2.

2. The compound of formula I according to claim 1, wherein
i) A is -NO₂; or
i) X is O; Y is O; or
i) A is -NO₂, X is O; Y is O; or
ii) A is -NO₂, X is O; Y is O; and Z is N,
iii) A is -NO₂, X is O; Y is O; or
iv) A is -NO₂, X is O; Y is O; and Z is N, or
v) B is -CF₃, halogen, -CN, or propargyl,
or any combinations between i), ii), iii), iv) and v)

3. The compound of formula I according to claim 1 or 2, wherein the compound is selected from the group consisting in 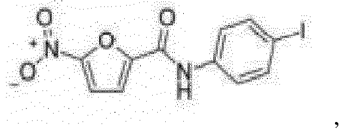 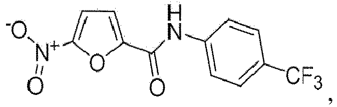 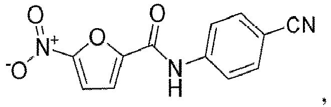 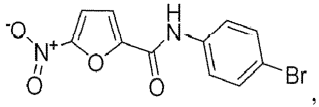 and

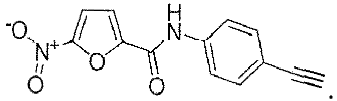

4. A compound having formula I according to anyone of the preceding claims, or a racemates, enantiomer, diastereomer and pharmaceutically acceptable salt thereof, for use in the treatment of a STING associated disease.

5. The compound according to claim 5, wherein the STING associated disease is selected from the group comprising a cancer, a neurological disorder, an autoimmune disease, hepatitis B, uveitis, cardiovascular disease, age-related macular degeneration and mucositis.

6. A pharmaceutical composition comprising a compound of anyone of claims 1 to 4, or a pharmaceutically acceptable salt thereof, and a pharmaceutically acceptable excipient.

7. Use of a compound having formula I according to anyone of the claims 1 to 4, or pharmaceutical composition of claim 6, in the manufacture of a medicament.

8. The use according to claim 7, wherein the medicament is for the treatment of a STING associated disease.

9. A compound that inhibits the signaling molecule STING by binding at least one amino acid of a region including the amino acid residues 85 to 95.

10. The compound according to claim 9, wherein the binding is a covalent binding.

11. The compound according to claim 9, wherein the compound covalently binds to the transmembrane-located cysteine C91.

12. A kit comprising (1) a composition comprising a pharmaceutically effective amount of a compound of any one of claims 1 to 3 or a racemate, enantiomer, diastereomer and pharmaceutically acceptable salts thereof, and (2) instructions for use.


SEQUENCE LISTING

<110>  ECOLE POLYTECHNIQUE FEDERALE DE LAUSANNE (EPFL)

<120>  STING inhibitors

<130>  51040/EP

<160>  13

<170>  PatentIn version 3.5

<210>  1
<211>  90
<212>  DNA
<213>  Artificial Sequence

<220>
<223>  synthetique DNA

<400>  1
tacagatcta ctagtgatct atgactgatc tgtacatgat ctacatacag atctactagt      60

gatctatgac tgatctgtac atgatctaca                                        90


<210>  2
<211>  19
<212>  DNA
<213>  Artificial Sequence

<220>
<223>  synthetique DNA

<400>  2
gtcatcccag agctgaacg                                                    19


<210>  3
<211>  21
<212>  DNA
<213>  Artificial Sequence

<220>
<223>  synthetique DNA

<400>  3
tcatacttgg caggtttctc c                                                 21


<210>  4
<211>  20
<212>  DNA
<213>  Artificial Sequence

<220>
<223>  synthetique DNA

<400>  4
ctccagctcc aagaaaggac                                                   20


<210>  5

<211>  20
<212>  DNA
<213>  Artificial Sequence

<220>
<223>  synthetique DNA

<400>  5
tggcaaaggc agtgtaactc                                                    20


<210>  6
<211>  20
<212>  DNA
<213>  Artificial Sequence

<220>
<223>  synthetique DNA

<400>  6
aaggaccaga tcacggacac                                                    20


<210>  7
<211>  20
<212>  DNA
<213>  Artificial Sequence

<220>
<223>  synthetique DNA

<400>  7
catcctccag ggttttcaga                                                    20


<210>  8
<211>  20
<212>  DNA
<213>  Artificial Sequence

<220>
<223>  synthetique DNA

<400>  8
aagaagcaga ttgcccagaa                                                    20


<210>  9
<211>  20
<212>  DNA
<213>  Artificial Sequence

<220>
<223>  synthetique DNA

<400>  9
tctgcgtcag aaagacctca                                                    20


<210>  10
<211>  20
<212>  DNA
<213>  Artificial Sequence

<220>
<223>  synthetique DNA

<400>  10
gagtcaacgg atttggtcgt                                                    20


<210>  11
<211>  20
<212>  DNA
<213>  Artificial Sequence

<220>
<223>  synthetique DNA

<400>  11
gacaagcttc ccgttctcag                                                    20


<210>  12
<211>  20
<212>  DNA
<213>  Artificial Sequence

<220>
<223>  synthetique DNA

<400>  12
cagcatctgc tggttgaaga                                                    20


<210>  13
<211>  20
<212>  DNA
<213>  Artificial Sequence

<220>
<223>  synthetique DNA

<400>  13
cattacctga aggccaagga                                                    20